# EUROPÄISCHE PATENTSCHRIFT

(11) **EP 3 395 274 B1**
(45) Veröffentlichungstag und Bekanntmachung des Hinweises auf die Patenterteilung: **14.02.2024**
(21) Anmeldenummer: 18167954.9
(22) Anmeldetag: 18.04.2018
(51) Int. Cl.: B01F 33/501, B01F 35/71, B01F 35/75, A61B 17/88, B01F 101/20

(54) **KNOCHENZEMENTAPPLIKATIONSVORRICHTUNG MIT VERSCHLUSSMITTEL AM AUSTRAGSKOLBEN**
BONE CEMENT APPLICATION DEVICE WITH CLOSURE MEANS AT THE OUTPUT PISTON
DISPOSITIF D'APPLICATION DE CIMENT OSSEUX POURVU DE MOYEN DE FERMETURE SUR LE PISTON DE DISTRIBUTION

(30) Priorität: 28.04.2017 DE 102017109255
(43) Veröffentlichungstag der Anmeldung: 31.10.2018
(73) Patentinhaber: Heraeus Medical GmbH, 61273 Wehrheim (DE)
(72) Erfinder: Vogt, Sebastian, 99092 Erfurt (DE); Kluge, Thomas, 56179 Vallendar (DE)
(74) Vertreter: Schultheiss & Sterzel Patentanwälte PartG mbB

(56) Entgegenhaltungen:
- WO-A1-2011/089480
- US-A- 4 676 406
- US-A1- 2011 308 665
- US-A1- 2014 198 601
- US-B1- 6 367 962

## Beschreibung

Die Erfindung betrifft eine Vorrichtung zum Herstellen eines Knochenzementteigs aus einer Monomerflüssigkeit und einem Zementpulver als Ausgangskomponenten des Knochenzementteigs und zum Austragen des gemischten Knochenzementteigs.

Die Erfindung betrifft auch ein Verfahren zur Herstellung eines Knochenzementteigs, insbesondere eines pastenförmigen Polymethylmethacrylat-Knochenzementteigs, mit einer Vorrichtung.

Gegenstand der Erfindung ist insbesondere eine Vorrichtung zum separaten Lagern des Zementpulvers und der Monomerflüssigkeit von Polymethylmethacrylat-Knochenzement, zum anschließenden Vermischen des Zementpulvers mit der Monomerflüssigkeit zur Bildung eines Knochenzementteigs und zum Austragen des gemischten Knochenzementteigs. Es handelt sich bei der erfindungsgemäßen Vorrichtung bevorzugt um ein Full-Prepacked-Zementiersystem.

Polymethylmethacrylat-(PMMA)-Knochenzemente gehen auf die grundlegenden Arbeiten von Sir Charnley zurück (Charnley, J.: Anchorage of the femoral head prosthesis of the shaft of the femur. J. Bone Joint Surg. 42 (1960) 28-30.). Konventionelle Polymethylmethacrylat-Knochenzemente (PMMA-Knochenzemente) sind aus einer pulverförmigen Komponente und einer flüssigen Monomerkomponente zusammengesetzt (K.-D. Kühn: Knochenzemente für die Endoprothetik: Ein aktueller Vergleich der physikalischen und chemischen Eigenschaften handelsüblicher PMMA-Zemente. Springer-Verlag Berlin Heidelberg New York, 2001). Die Monomerkomponente enthält im Allgemeinen das Monomer Methylmethacrylat und einen darin gelösten Aktivator (N,N-Dimethyl-p-toluidin). Die Pulverkomponente, auch als Zementpulver oder Knochenzementpulver bezeichnet, weist ein oder mehrere Polymere auf, die auf Basis von Methylmethacrylat und Comonomeren, wie Styren, Methylacrylat oder ähnlichen Monomeren durch Polymerisation, vorzugsweise Suspensionspolymerisation, hergestellt sind, einen Röntgenopaker und den Initiator Dibenzoylperoxid auf. Beim Vermischen der Pulverkomponente mit der Monomerkomponente entsteht durch Quellung der Polymere der Pulverkomponente im Methylmethacrylat ein plastisch verformbarer Teig, der eigentliche Knochenzement beziehungsweise Knochenzementteig. Beim Vermischen der Pulverkomponente mit der Monomerkomponente reagiert der Aktivator N,N-Dimethyl-p-toluidin mit Dibenzoylperoxid unter Bildung von Radikalen. Die gebildeten Radikale initiieren die radikalische Polymerisation des Methylmethacrylats. Mit fortschreitender Polymerisation des Methylmethacrylats erhöht sich die Viskosität des Knochenzementteigs, bis dieser erstarrt. PMMA-Knochenzemente können in geeigneten Mischbechern mit Hilfe von Spateln durch Vermischen des Zementpulvers mit der Monomerflüssigkeit vermischt werden. Dabei kann es zum Einschluss von Luftblasen im Knochenzementteig kommen, welche die mechanischen Eigenschaften des ausgehärteten Knochenzements negativ beeinflussen können.

Zur Vermeidung von Lufteinschlüssen im Knochenzementteig wurden eine Vielzahl von Vakuum-Zementiersystemen beschrieben, von denen exemplarisch folgende genannt sind: US 6 033 105 A, US 5 624 184 A, US 4 671 263 A, US 4 973 168 A, US 5 100 241 A, WO 99/67015 A1, EP 1 020 167 A2, US 5 586 821 A, EP 1 016 452 A2, DE 36 40 279 A1, WO 94/26403 A1, EP 1 005 901 A2, EP 1 886 647 A1, US 5 344 232 A.

Eine Weiterentwicklung in der Zementiertechnik stellen Zementiersysteme dar, in denen sowohl das Zementpulver als auch die Monomerflüssigkeit bereits in separaten Kompartimenten der Mischvorrichtungen verpackt sind und die erst unmittelbar vor der Zementapplikation im Zementiersystem miteinander vermischt werden. Solche geschlossenen Full-Prepacked-Mischvorrichtungen wurden mit der EP 0 692 229 A1, der DE 10 2009 031 178 B3, der US 5 997 544 A, der US 6 709 149 B1, der DE 698 12 726 T2, der EP 0 796 653 A2 und der US 5 588 745 A vorgeschlagen.

Das Patent DE 10 2009 031 178 B3 offenbart eine Lager- und Mischvorrichtung als Full-Prepacked-Mischvorrichtung, in dem die zur Herstellung des Knochenzementteigs notwendigen Ausgangskomponenten bereits in der Lager- und Mischvorrichtung gelagert sind und in der Lager- und Mischvorrichtung zusammengeführt und gemischt werden können. Die Lager- und Mischvorrichtung weist einen zweiteiligen Austragskolben zum Verschluss einer Zementkartusche auf. Dabei wird eine Kombination aus einem gasdurchlässigen Sterilisationskolben und einem gasundurchlässigen Dichtungskolben verwendet.

Polymethylmethacrylat-Knochenzemente werden nach Vermischung des Zementpulvers mit der flüssigen Monomerkomponente im noch nicht ausgehärteten, pastenförmigen Zustand als Knochenzementteig appliziert. Bei Verwendung von Mischvorrichtungen befindet sich der Knochenzementteig im Fall von Pulver-Flüssigkeits-Zementen in einer Kartusche. Bei der Applikation solcher konventioneller PMMA-Knochenzemente, wird nach der Vermischung der beiden Ausgangskomponenten der gebildete Knochenzementteig mit Hilfe von manuell bedienbaren Auspressvorrichtungen ausgepresst. Aus der Kartusche wird der Knochenzementteig durch Bewegung eines Austragskolbens herausgedrückt. Die Austragskolben haben üblicherweise einen Durchmesser zwischen 30 mm bis 40 mm und damit an der Außenseite, an welcher ein Stößel beziehungsweise eine Stange der Auspressvorrichtung beim Auspressvorgang angreift, eine Fläche von 7,0 cm² bis 12,5 cm². Die Bewegung des Austragskolbens wird bevorzugt durch manuell bedienbare, mechanische Auspressvorrichtungen bewirkt. Diese manuellen Auspressvorrichtungen erreichen normalerweise eine Auspresskraft im Bereich von ungefähr 1,5 kN bis 3,5 N.

Diese einfachen mechanischen Auspressvorrichtungen nutzen insbesondere Klemmstangen zum Auspressen, die durch einen manuell zu betätigenden Kipphebel angetrieben werden. Die manuell angetriebenen Auspressvorrichtungen sind seit Jahrzehnten weltweit erprobt und stellen bisher den Stand der Technik dar. Vorteilhaft an diesen Auspressvorrichtungen ist, dass der medizinische Anwender über die aufzubringende Handkraft ein Gefühl für den Eindringwiderstand des Knochenzementteigs in die Knochenstrukturen (Spongiosa) hat.

Bei der Verwendung aller bisher bekannten Full-Prepacked-Mischvorrichtungen muss der medizinische Anwender mehrere Arbeitsschritte in einer vorbestimmten Reihenfolge an den Vorrichtungen nacheinander durchführen bis der gemischte Knochenzementteig vorliegt und appliziert werden kann. Eine Verwechslung der Arbeitsschritte kann zum Versagen der Mischvorrichtung führen und daher Störungen im OP-Ablauf verursachen. Kostenintensive Schulungen der medizinischen Anwender sind deshalb notwendig, um Anwenderfehler zu vermeiden.

In der WO 00/35506 A1 wird eine Vorrichtung vorgeschlagen, bei der Polymethylmethacrylat-Zementpulver in einer Kartusche gelagert wird, wobei das Zementpulver das gesamte Volumen der Kartusche ausfüllt und die Zwischenräume zwischen den Partikeln des Zementpulvers ein solches Volumen hat, das dem Volumen der Monomerflüssigkeit entspricht, das zur Herstellung von Knochenzementteig mit dem in der Kartusche gelagerten Zementpulver notwendig ist. Diese Vorrichtung ist so aufgebaut, dass durch Vakuumeinwirkung die Monomerflüssigkeit von oben in die Kartusche eingeleitet wird, wobei hierzu ein Vakuum an einem Vakuumanschluss an der Unterseite der Kartusche angelegt wird. Dadurch wird die Monomerflüssigkeit durch das Zementpulver gezogen, wobei die in den Zwischenräumen der Zementpulverpartikel befindliche Luft durch die Monomerflüssigkeit verdrängt wird. Auf eine mechanische Durchmischung des gebildeten Zementteigs mit einem Rührer wird dabei verzichtet.

Nachteilig an diesem System ist, dass Zementpulver, die schnell mit der Monomerflüssigkeit anquellen, mit dieser Vorrichtung nicht angemischt werden können, weil die schnell anquellenden Zementpulverpartikel nach einem Eindringen der Monomerflüssigkeit in das Zementpulver von ungefähr 1 bis 2 cm eine gelartige Barriere bilden und die Migration der Monomerflüssigkeit durch das gesamte Zementpulver behindern. Konventionelle Zementpulver zeigen zudem das Phänomen, dass auf Grund der unterschiedlichen Oberflächenenergien die Zementpulverpartikel durch Methylmethacrylat nur schlecht benetzt werden. Dadurch dringt das Methylmethacrylat nur relativ langsam in das Zementpulver ein. Weiterhin kann bei Vakuumeinwirkung nicht ausgeschlossen werden, dass nach vollständiger Durchdringung des Zementpulvers durch die Monomerflüssigkeit die Monomerflüssigkeit über den Vakuumanschluss abgesaugt wird. Dann steht nicht genügend Monomerflüssigkeit für die Aushärtung durch radikalische Polymerisation zur Verfügung beziehungsweise das Mischungsverhältnis wird ungewollt verändert und damit auch die Konsistenz des Knochenzementteigs. Weiterhin ist es problematisch, dass die zwischen den Zementpulverpartikeln eingeschlossen Luft durch die Monomerflüssigkeit von oben nach unten verdrängt werden soll, weil die gegenüber der Monomerflüssigkeit spezifisch leichtere Luft auf Grund der Schwerkraft das Bestreben hat, im Zementpulver nach oben zu wandern und nicht nach unten in Richtung Vakuumanschluss zu migrieren.

Aus dem Kleb- und Dichtstoffbereich sind auch elektrisch angetriebene Auspressvorrichtungen bekannt. Diese Vorrichtungen können sowohl mit Akkumulatoren und mit Batterien als auch mit Hilfe einer stationären Stromversorgung angetrieben werden. Diese Vorrichtungen können mit ihren zum Teil sehr großen Auspresskräften besonders zähe pastenförmige Massen auspressen. Nachteilig ist jedoch bei der Verwendung von Elektromotoren, dass diese Buntmetalle enthalten und kostenintensiv in der Beschaffung sind. Im steril zu haltenden OP-Bereich müssen derartige Vorrichtungen aufwendig sterilisiert oder sogar ausgetauscht werden. Bei einer elektrischen Verkabelung kann die Bewegung des Anwenders im OP behindert werden.

Weiterhin wurden auch pneumatische Vorrichtungen vorgeschlagen. Diese Geräte erfordern einen stationären oder mobilen Druckluftanschluss (US 2 446 501 A, DE 20 2005 010 206 U1). Dazu sind Druckluftschläuche notwendig, welche die Bewegung des Anwenders behindern können.

Alternativ dazu ist auch die Verwendung von Druckgaspatronen zur Bereitstellung von Druckgas möglich. Dazu wurden Vorrichtungen vorgeschlagen, bei denen der Druckgaszufluss durch ein Ventil und zusätzlich durch ein zweites Ventil der Strom der viskosen Masse gesteuert werden (US 2004/0074927 A1, US 6 935 541 B1). Bei diesen Vorrichtungen sind die Gaspatronen in den Vorrichtungen integriert. Bei derartigen an Druckluft angeschlossenen oder Druckgaspatronen enthaltenden Systemen ist immer eine Druckgasquelle erforderlich, ohne die die Systeme nicht mehr anwendbar sind.

Die WO 2011/089 480 A1 offenbart eine Vorrichtung und ein Verfahren, die der Erfindung ähneln, jedoch ohne einen Zapfen als Verschlussmittel und ohne ein Treffen des Austragskolbens auf die Vorderseite der Kartusche. Sie offfenbart eine Vorrichtung zum Mischen von Knochenzement, bei der ein Mischer im Innenraum einer Kartusche vorgesehen ist, mit dem der Inhalt der Kartusche manuell zu mischen ist.

In der nicht vorveröffentlichten DE 10 2016 121 607 A1 wurde ein Full-Prepacked-Mischsystem mit einer Kartusche enthaltend ein Knochenzementpulver vorgeschlagen. In der Kartusche ist ein Austragskolben vorgesehen und hinter der Kartusche eine Aufnahme enthaltend einen Monomerflüssigkeitsbehälter angeordnet. An der Rückseite der Aufnahme befindet sich ein Förderkolben, mit dem der Monomerflüssigkeitsbehälter zerquetscht werden kann und die Monomerflüssigkeit aus der Aufnahme in die Kartusche gepresst werden kann.

Es zeigte sich in praktischen Versuchen, dass der mit dieser Vorrichtung hergestellte Knochenzementteig bei der Verwendung eines geeigneten Zementpulvers immer eine gute Konsistenz besitzt. Wird der geborstene Monomerflüssigkeitsbehälter beim Monomertransfer maximal komprimiert, dann erhält man reproduzierbar einen guten Zementteig. Es kann jedoch bei bestimmten Konstellationen zu einer ungewünschten Veränderung der Konsistenz des Knochenzementteigs am Ende des Auspressvorgangs kommen, bei dem sich das Mischungsverhältnis zwischen dem Zementpulver und der Monomerflüssigkeit verändert hat.

Im Rahmen der vorliegenden Erfindung wurde gefunden, dass dies mit der Wahl und der Stabilität des Monomerflüssigkeitsbehälters zusammenhängt. Bei unvollständiger Kompression des geborstenen Monomerflüssigkeitsbehälters, die zum Beispiel durch die Wahl eines Monomerflüssigkeitsbehälters mit sehr stabilen Wandungen auftreten kann, kann nämlich ein Rest der Monomerflüssigkeit zwischen dem Austragskolben und dem Förderkolben innerhalb der Fragmente des geborstenen Monomerflüssigkeitsbehälters verbleiben, der am Ende des Auspressens des Knochenzementteigs durch eine anschließende Nachkompression des geborstenen Monomerflüssigkeitsbehälters infolge einer axialen Bewegung des Förderkolbens in Richtung des Austragskolbens durch das Austragsrohr austreten kann.

Die Aufgabe der vorliegenden Erfindung besteht also darin, die Nachteile des Stands der Technik zu überwinden. Insbesondere besteht die Aufgabe der Erfindung in der Entwicklung einer Vorrichtung die zum Mischen des Knochenzementteigs aus den Ausgangskomponenten und zum Austragen des gemischten Knochenzementteigs vorgesehen und geeignet ist sowie eines Verfahrens zur Herstellung eines Knochenzementteigs, insbesondere eines pastenförmigen Polymethylmethacrylat-Knochenzementteigs, wobei der Knochenzementteig aus einem Zementpulver und einer Monomerflüssigkeit mit einer solchen Vorrichtung hergestellt wird mit dem die Nachteile der bisherigen Vorrichtungen und Verfahren überwunden werden. Die Erfindung hat die Aufgabe eine solche Vorrichtung derart zu verbessern, dass auch bei einer nicht vollständigen Kompression des Monomerflüssigkeitsbehälters am Ende des Austrags des Knochenzementteigs ein Austritt der Monomerflüssigkeit aus dem Austragsrohr der Kartusche wirksam verhindert wird. Mit der erfindungsgemäßen Vorrichtung und dem erfindungsgemäßen Verfahren soll also erreicht werden, dass auch bei einem sehr einfachen und kostengünstigen Aufbau der Vorrichtung und bei gleichzeitig sehr einfacher und unkomplizierter Anwendbarkeit der Vorrichtung von Anfang bis Ende des Auspressvorgangs ein homogener Knochenzementteig erzeugt und appliziert werden kann.

Die Vorrichtung soll durch eine einfache Auspressvorrichtung anzutreiben sein und dabei möglichst einfach in der Bedienung sein. Der Aufbau soll kostengünstig sein, damit die Vorrichtung aus hygienischen Gründen nur einmalig verwendet werden kann. Es sollen möglichst viele oder alle in der Vorrichtung ablaufenden Prozesse, wie die Vermischung der Ausgangskomponenten, das Austragen des Knochenzementteigs und gegebenenfalls auch das Öffnen des Monomerflüssigkeitsbehälters und gegebenenfalls auch das Öffnen der Kartusche mit möglichst wenigen Arbeitsschritten und so weit als möglich automatisiert ablaufen und vorzugsweise mit einem einzigen linearen Antrieb anzutreiben sein.

Die Aufgabe der Erfindung besteht also auch in der Entwicklung einer Vorrichtung zum Vermischen von Zementpulver und Monomerflüssigkeit. Die Handhabung der Vorrichtung soll maximal vereinfacht sein, um Anwendungsfehler infolge von fehlerhaft durchgeführten Montageschritten grundsätzlich zu vermeiden. Der medizinische Anwender soll die Vorrichtung nach der Entnahme aus einer Verpackung mit einer Auspressvorrichtung verbinden und diese anschließend betätigen. Weitere Montage- und Arbeitsschritte sollen durch den Aufbau der Vorrichtung vermieden werden. Die Vorrichtung soll vorzugsweise auch eine sichere Lagerung von Zementpulver und Monomerflüssigkeit in voneinander getrennten Kompartimenten ermöglichen, so dass während der Lagerung der Vorrichtung eine unbeabsichtigte Vermischung der Zementkomponenten ausgeschlossen ist. Die Vorrichtung soll eine Sterilisation mit dem Gas Ethylenoxid ermöglichen. Das in der Vorrichtung gelagerte Zementpulver muss dazu für Ethylenoxid zugänglich sein. Die Vorrichtung soll mit Hilfe einer im OP manuell angetriebenen Auspressvorrichtung aktivierbar sein, so dass nach der form- oder kraftschlüssigen Verbindung der Vorrichtung mit der Auspressvorrichtung durch Betätigung der Auspressvorrichtung die axial vortreibbare Stange der Auspressvorrichtung auf die Vorrichtung einwirkt, gegebenenfalls den Monomerflüssigkeitsbehälter öffnet und anschließend bei weiterer Bewegung der Stange die Monomerflüssigkeit in das Zementpulver transferiert. Die Vermischung der Monomerflüssigkeit mit dem Zementpulver soll ohne einen von außen manuell zu bewegenden Mischer erfolgen. Es soll möglichst nur mit der Vorwärtsbewegung der Stange der Auspressvorrichtung die Vermischung der Zementkomponenten unter Bildung des Knochenzementteigs und das Auspressen des gemischten Knochenzementteigs vorgenommen werden. Bevorzugt soll möglichst nur mit der Vorwärtsbewegung der Stange der Auspressvorrichtung auch das Öffnen des Monomerflüssigkeitsbehälters und der nachfolgende Monomerflüssigkeitstransfer in das Zementpulver vorgenommen werden.

Bevorzugt soll der zu entwickelnde Knochenzement-Applikator keine zwei miteinander verbundenen und synchron vorzutreibenden Stangen erfordern, damit die gesamte Vorrichtung nicht wesentlich aufwendiger, länger und größer wird als die bisher für die konventionellen Pulver-Flüssigkeits-Polymethylmethacrylat-Knochenzemente üblichen Mischvorrichtungen. Es soll eine einfache Lösung gefunden werden, die es erlaubt, möglichst mit einer Auspressvorrichtung, die nur eine Stange und gegebenenfalls einen daran befestigten Teller aufweist, den Knochenzementteig auszutreiben.

Die Aufgaben der Erfindung werden gelöst durch eine Vorrichtung zum Herstellen eines Knochenzementteigs aus einer Monomerflüssigkeit und einem Zementpulver als Ausgangskomponenten des Knochenzementteigs und zum Austragen des gemischten Knochenzementteigs, die Vorrichtung aufweisend eine Kartusche mit einem zylindrischen Innenraum, wobei der Innenraum der Kartusche an der Vorderseite bis auf eine Austragsöffnung zum Austreiben des Knochenzementteigs verschlossen ist, wobei im Innenraum der Kartusche ein in Richtung der Austragsöffnung drückbarer Austragskolben angeordnet ist und wobei das Zementpulver im Innenraum der Kartusche zwischen der Austragsöffnung und dem Austragskolben angeordnet ist, wobei an der der Austragsöffnung zugewandten Vorderseite des Austragskolbens ein Verschlussmittel angeordnet ist, das die Austragsöffnung verschließt, wenn der Austragskolben gegen die Vorderseite des Innenraums der Kartusche gedrückt ist, wobei das Verschlussmittel ein aus der Vorderseite des Austragskolbens vorspringender Zapfen ist, der zumindest teilweise in die Austragsöffnung einschiebbar ist, um die Austragsöffnung zu verschließen, wobei der Zapfen eine weitere Bewegung des Austragskolbens in Richtung der Vorderseite der Kartusche blockiert, so dass der Austragskolben zumindest bereichsweise von der Vorderseite des Innenraums der Kartusche beabstandet ist und ein Totvolumen in dem Innenraum der Kartusche verbleibt, wenn der Austragskolben gegen die Vorderseite des Innenraums der Kartusche gedrückt ist.

Bevorzugt ist die erfindungsgemäße Vorrichtung auch zum Lagern des Zementpulvers vorgesehen und besonders bevorzugt auch zum Lagern der Monomerflüssigkeit vorgesehen. Es kann vorgesehen sein, dass die Austragsöffnung in der Vorderseite der Kartusche angeordnet ist. Bevorzugt wird ist an der Vorderseite der Kartusche ein Austragsrohr angeordnet, dass die Austragsöffnung begrenzt.

Dass ein Totvolumen im Innenraum der Kartusche verbleibt, bedeutet, dass zwischen der durch das Verschlussmittel geschlossenen Austragsöffnung und dem Austragskolben ein Volumen verbleibt, das mit einer Mischung aus dem Zementpulver und der Monomerflüssigkeit gefüllt ist, wenn der Austragskolben gegen die Vorderseite des Innenraums der Kartusche gedrückt ist.

Dass des Austragskolben gegen die Vorderseite des Innenraumus der Kartsche gedrückt ist, bedeutet nicht, dass der Austragskolben in unmittelbarem Kontakt mit der Vorderseite des Innenraums ist. Stattdessen ist das Verschlussmittel in direktem Kontakt mit der Vorderseite des Innenraums, wenn der Austragskolben gegen die Vorderseite des Innenraums der Kartusche gedrückt ist.

Die Kartusche ist vorzugsweise aus einem thermoplastischen Kunststoff gefertigt, insbesondere mit einem Spritzgussverfahren.

Der Innenraum der Kartusche hat eine zylindrische Geometrie. Die zylindrische Form ist die einfachste, mit der sich der Innenraum der Kartusche realisieren lässt. Unter einer zylindrischen Form ist geometrisch die Form eines allgemeinen Zylinders mit einer beliebigen Grundfläche zu verstehen, also nicht nur ein Zylinder mit einer kreisförmigen Grundfläche. Die Innenwand des Innenraums der Kartusche kann also durch den Zylindermantel eines Zylinders mit beliebiger Grundfläche realisiert sein, insbesondere mit unterschiedlicher Grundfläche realisiert sein, das heißt auch mit nicht kreisförmigen oder nicht runden Grundflächen. Erfindungsgemäß wird jedoch eine zylindrische Geometrie mit rotationssymmetrischer und insbesondere kreisrunder Grundfläche für den Innenraum bevorzugt, da diese am einfachsten zu fertigen ist.

Es ist mit der vorliegenden Erfindung auch vorgesehen, dass das Verschlussmittel ein aus der Vorderseite des Austragskolbens vorspringender Zapfen ist.

Hierdurch wird sichergestellt, dass mit dem Verschlussmittel eine Kraft auf die Austragsöffnung bewirkt werden kann, die zur Abdichtung der Austragsöffnung nutzbar ist. Zudem kann dadurch auch die Beabstandung des Austragskolbens von der Vorderseite des Innenraums der Kartusche gewährleistet werden.

Bevorzugt ist die Vorderseite des Austragskolbens bis auf das Verschlussmittel eben.

Vorzugsweise ist der Innenraum der Kartusche an seiner Vorderseite durch einen Kartuschenkopf begrenzt, in dem die Austragsöffnung angeordnet ist. Ebenfalls kann bevorzugt vorgesehen sein, dass am Kartuschenkopf ein Austragsrohr angeordnet ist, das die Austragsöffnung verlängert.

Des Weiteren kann vorgesehen sein, dass das Totvolumen zumindest 1 cm³ groß ist, bevorzugt zumindest 3 cm³ groß ist.

Diese Totvolumina sind ausreichend, um nicht vollständig gemischte Anteile des Knochenzementteigs, die im Bereich des Austragskolbens in dem Innenraum der Kartusche entstehen können, im Innenraum der Kartusche zurückzuhalten. Dadurch kann verhindert werden, dass am Ende des Austragsvorgangs schlecht gemischter Knochenzementteig oder ein Knochenzementteig mit sich ändernder Zusammensetzung und damit Konsistenz ausgetragen wird, der nicht verwendbar ist.

Ferner kann vorgesehen sein, dass das Verschlussmittel abdichtend ist, insbesondere bei einem auf der Rückseite des Austragskolbens wirkenden Druck.

Hiermit wird erreicht, dass auch keine geringen Mengen des im Bereich des Austragskolbens vorhandenen schlecht gemischten Knochenzementteigs durch die Austragsöffnung vordringen können.

Bevorzugt kann auch vorgesehen sein, dass der Zapfen eine geneigte, insbesondere konische, Dichtfläche aufweist, die gegen eine umlaufende Dichtkante oder Dichtfläche der Austragsöffnung abdichtet, wenn der Austragskolben gegen die Vorderseite des Innenraums der Kartusche gedrückt ist, oder die Austragsöffnung eine geneigte, insbesondere konische, Dichtfläche aufweist, die gegen eine umlaufende Dichtkante oder Dichtfläche des Verschlussmittels abdichtet, wenn der Austragskolben gegen die Vorderseite des Innenraums der Kartusche gedrückt ist.

Derartige Abdichtungen sind einfach zu realisieren und entfalten bei rückseitigem Druck eine hohe Dichtwirkung. Dies gilt insbesondere bei geeigneter Wahl der Materialien der Dichtflächen und der Dichtkanten. Vorzugsweise sollten also die Dichtkanten die gleiche Härte haben wie die Dichtflächen oder eine größere Härte haben als die Dichtflächen.

Der Durchmesser der Basis der geneigten beziehungsweise konischen Dichtfläche des Zapfens hat hierzu einen größeren Durchmesser als der Innendurchmesser der Austragsöffnung, oder der Durchmesser der Basis der geneigten beziehungsweise konischen Dichtfläche der Austragsöffnung hat hierzu einen größeren Durchmesser als der Innendurchmesser der Dichtkante des Zapfens. An der Spitze haben die geneigten beziehungsweise konischen Dichtflächen einen kleineren Durchmesser als die korrespondierenden Dichtkanten, damit der Zapfen beziehungsweise ein in den Innenraum der Kartusche ragender Stutzen an der Austragsöffnung leicht eingeführt werden kann.

Es ist vorgesehen, dass das Verschlussmittel ein Zapfen ist, der zumindest teilweise in die Austragsöffnung einschiebbar ist, um die Austragsöffnung zu verschließen.

Hiermit wird erreicht, dass die Dichtwirkung bei weiterem Vortreiben des Austragskolbens beziehungsweise bei weiter steigendem Druck auf die Rückseite des Austragskolbens erhöht wird. Dies bewirkt, dass die Austragsöffnung umso besser abgedichtet wird, je tiefer oder je fester der Zapfen in die Austragsöffnung eingeschoben wird. Zudem ist ein solches über den rückseitigen Druck auf den Austragskolben dicht schließendes System leicht und kostengünstig aufzubauen.

Es kann auch vorgesehen sein, dass in dem Austragskolben zumindest eine für die Monomerflüssigkeit und Gase durchlässige aber für das Zementpulver undurchlässige Verbindung vorgesehen ist, die die Vorderseite des Austragskolbens mit der Rückseite des Austragskolbens verbindet.

Hiermit wird verhindert, dass das Zementpulver in die Verbindung im Austragskolben eindringt, dort mit der Monomerflüssigkeit reagiert und beim Anquellen des Zementpulvers in der Verbindung die Verbindung verschlossen wird. Gleichzeitig kann die Monomerflüssigkeit durch die Verbindung im Austragskolben in den Innenraum der Kartusche eingeleitet werden.

Hierzu ist vorzugsweise ein für die Monomerflüssigkeit und für Gase durchlässiger Filter, insbesondere ein Porenfilter, an der Einmündung der zumindest einen Verbindung in den Innenraum der Kartusche angeordnet.

Es kann ferner vorgesehen sein, dass die zumindest eine Verbindung im Austragskolben innerhalb des Zapfens in den Innenraum der Kartusche mündet, wobei vorzugsweise die zumindest eine Verbindung durch mehrere radiale Bohrungen in der Mantelfläche des Zapfens in den Innenraum der Kartusche mündet.

Dadurch wird sichergestellt, dass die Monomerflüssigkeit in einem zentralen Bereich in den Innenraum der Kartusche und damit in das Zementpulver fließt und dadurch weniger leicht an der Wandung des Innenraums der Kartusche und an dem Zementpulver vorbei in Richtung der Austragsöffnung fließen kann. Damit wird ein besser durchmischter Knochenzementteig erreicht.

Die Verbindung kann in Form von konzentrisch um das Verschlussmittel angeordneten Durchtrittsöffnungen in den Innenraum der Kartusche münden.

Besonders bevorzugt kann vorgesehen sein, dass die Vorrichtung eine Aufnahme aufweist, in der die Monomerflüssigkeit, insbesondere ein Monomerflüssigkeitsbehälter enthaltend die Monomerflüssigkeit, enthalten ist, wobei die Rückseite der Kartusche mit der Vorderseite der Aufnahme verbunden ist, vorzugsweise derart verbunden ist, dass der Innenraum der Kartusche mit dem Innenraum der Aufnahme fluchtet.

Hierdurch ist die Vorrichtung auch zum Lagern der Monomerflüssigkeit geeignet sowie zum Mischen der Monomerflüssigkeit mit dem Zementpulver innerhalb der Vorrichtung. Die Vorrichtung ist damit ein Full-Prepacked-Mischsystem.

Die Aufnahme ist vorzugsweise aus einem thermoplastischen Kunststoff gefertigt, insbesondere mit einem Spritzgussverfahren.

Hierdurch kann die Vorrichtung kostengünstig als hygienisches Wegwerfprodukt gefertigt werden.

Bei erfindungsgemäßen Vorrichtungen, bei denen die Monomerflüssigkeit in einem Monomerflüssigkeitsbehälter innerhalb der Vorrichtung angeordnet ist, kann vorgesehen sein, dass der Monomerflüssigkeitsbehälter eine Glasampulle, eine Plastikampulle, ein Kunststofffolienbeutel oder ein Aluminium-Kunststoff-Verbund-Beutel ist. In derartigen Monomerflüssigkeitsbehältern kann die Monomerflüssigkeit besonders lange gelagert werden.

Bevorzugt kann auch vorgesehen sein, dass ein Innenraum der Aufnahme und der Innenraum der Kartusche über eine für die Monomerflüssigkeit und für Gase durchlässige aber für das Zementpulver undurchlässige Verbindung miteinander verbunden sind.

Hiermit wird gestellt, dass das Zementpulver nicht durch die Verbindung in den Innenraum der Aufnahme vordringt, dort vorzeitig mit der Monomerflüssigkeit reagiert und anschließend den Monomertransfer in den Innenraum der Kartusche verhindert. Die Verbindung ist besonders bevorzugt im Austragskolben angeordnet.

Des Weiteren kann vorgesehen sein, dass die Aufnahme einen zylindrischen Innenraum aufweist, in dem die Monomerflüssigkeit, insbesondere ein Monomerflüssigkeitsbehälter enthaltend die Monomerflüssigkeit, angeordnet ist.

Der Innenraum der Aufnahme hat eine zylindrische Geometrie. Die zylindrische Form ist die einfachste, mit der sich der Innenraum der Aufnahme realisieren lässt. Unter einer zylindrischen Form ist geometrisch die Form eines allgemeinen Zylinders mit einer beliebigen Grundfläche zu verstehen, also nicht nur ein Zylinder mit einer kreisförmigen Grundfläche. Die Innenwand des Innenraums der Aufnahme kann also durch den Zylindermantel eines Zylinders mit beliebiger Grundfläche realisiert sein, insbesondere mit unterschiedlicher Grundfläche sein, das heißt auch mit nicht kreisförmigen oder nicht runden Grundflächen. Erfindungsgemäß wird jedoch eine zylindrische Geometrie mit rotationssymmetrischer und insbesondere kreisrunder Grundfläche den Innenraum bevorzugt, da diese am einfachsten zu fertigen ist.

Ferner kann vorgesehen sein, dass in der Aufnahme ein in Längsrichtung der Aufnahme beweglicher Förderkolben angeordnet ist, der von einer Rückseite der Aufnahme aus in Richtung der Vorderseite vortreibbar ist, wobei die Monomerflüssigkeit, insbesondere ein Monomerflüssigkeitsbehälter enthaltend die Monomerflüssigkeit, zwischen dem Förderkolben und dem Austragskolben angeordnet ist.

Hiermit wird ein Full-Prepacked-Mischsystem bereitgestellt, in dem alle Ausgangskomponenten des Knochenzementteigs, nämlich die Monomerflüssigkeit und das Zementpulver enthalten sind und dort auch gelagert werden können.

Der Förderkolben schließt die Aufnahme an deren Rückseite flüssigkeitsdicht ab.

Bei erfindungsgemäßen Vorrichtungen mit Förderkolben kann alternativ oder zusätzlich vorgesehen sein, dass der Monomerflüssigkeitsbehälter im Inneren der Aufnahme durch eine Bewegung des Förderkolbens in Richtung der Vorderseite der Aufnahme zu öffnen ist, bevorzugt aufzubrechen oder aufzureißen ist.

Hierdurch wird erreicht, dass durch die axiale lineare Bewegung des Förderkolbens der Monomerflüssigkeitsbehälter geöffnet werden kann. Es kann dadurch eine Auspressvorrichtung mit nur einer Stange als axialer linearen Antrieb verwendet werden, um sowohl den Monomerflüssigkeitsbehälter zu öffnen als auch die Monomerflüssigkeit in die Kartusche zu pressen und auch den Knochenzementteig aus der Kartusche zu pressen.

Ferner kann bevorzugt auch vorgesehen sein, dass in der Wandung der Aufnahme zumindest eine Belüftungsöffnung angeordnet ist, die den Innenraum der Aufnahme mit der Umgebung verbindet.

Hierdurch kann der Innenraum der Aufnahme mit einem sterilisierenden Gas sterilisiert werden.

Gemäß einer bevorzugten Weiterbildung der vorliegenden Erfindung kann auch vorgesehen sein, dass die zumindest eine Belüftungsöffnung derart dicht im Bereich des Förderkolbens angeordnet ist, dass sie durch eine Bewegung des Förderkolbens in Richtung der Vorderseite der Aufnahme verschlossen wird, bevor ein in der Aufnahme angeordneter Monomerflüssigkeitsbehälter, in dem die Monomerflüssigkeit enthalten ist, durch die Bewegung des Förderkolbens geöffnet ist.

Hierdurch kann die Monomerflüssigkeit nicht aus dem Innenraum der Aufnahme austreten, wenn die zumindest eine Belüftungsöffnung von dem sich in Richtung der Vorderseite der Aufnahme bewegenden Förderkolben verschlossen wird, bevor der Monomerflüssigkeitsbehälter durch die Bewegung des Förderkolbens geöffnet wird, also beispielsweise von dem Förderkolben im Innenraum der Aufnahme zerdrückt, zersplittert oder aufgerissen wird.

Bevorzugt kann vorgesehen sein, dass die Aufnahme und die Kartusche einteilig durch einen röhrenförmigen Behälter gebildet sind.

Dieser Aufbau ist der einfachste und am kostengünstigsten zu realisierende Aufbau.

Bevorzugte Ausgestaltungen der vorliegenden Erfindung können sich auch dadurch auszeichnen, dass die Rückseite der Kartusche mit der Vorderseite der Aufnahme derart verbunden ist, dass der Innenraum der Kartusche mit dem Innenraum der Aufnahme fluchtet.

Hierdurch kann sichergestellt werden, dass zunächst der Förderkolben durch einen auf die Rückseite des Förderkolbens wirkenden Druck bewegt werden kann und anschließend der Förderkolben zum Antreiben des Austragskolbens genutzt werden kann, indem der Förderkolben gemeinsam mit dem Austragskolben weiter in Richtung der Austragsöffnung gedrückt wird.

Des Weiteren kann vorgesehen sein, dass an der Rückseite der Vorrichtung, ein Befestigungsmittel zur Befestigung einer Auspressvorrichtung angeordnet ist, mit der der Austragskolben in Richtung der Austragsöffnung drückbar ist.

Hiermit kann die Vorrichtung an einer Auspressvorrichtung mit einer vortreibbaren Stange angeschlossen und befestigt werden.

Ferner kann vorgesehen sein, dass an der Vorderseite des Förderkolbens zumindest eine vortretende Spitze, Kante und/oder Schneide zum Brechen des Monomerflüssigkeitsbehälters angeordnet ist.

Durch die Anwendung einer definierten Kraft an einer vorherbestimmten und räumlich begrenzten Stelle kann der Druck an dieser Stelle bei gleicher Kraft erhöht werden und so ein definiertes Brechen des Monomerflüssigkeitsbehälters erreicht werden. Dadurch wird der Ablauf des Aufbrechens des Monomerflüssigkeitsbehälters reproduzierbarer.

Es kann vorgesehen sein, dass das Zementpulver an der Vorderseite des Austragskolbens anliegt, insbesondere vollflächig anliegt, wobei vorzugsweise das Zementpulver in den Innenraum der Kartusche eingepresst ist.

Hierdurch wird verhindert, dass in der Kartusche größere Gaseinschlüsse verbleiben, die beim Mischen der Monomerflüssigkeit mit dem Zementpulver zu Gaseinschlüssen im Knochenzementteig führen könnten. Dies kann bei einem dicht geschütteten Zementpulver nicht passieren, da die Monomerflüssigkeit die Partikel des Zementpulvers gut benetzt und die Oberflächenspannung der Monomerflüssigkeit dann keine oder zumindest keine relevanten Gaseinschlüsse zwischen den Partikeln des Zementpulvers erlaubt.

Bevorzugt kann auch vorgesehen sein, dass die Austragsöffnung an deren Vorderseite mit einem Verschluss, insbesondere mit einem Stopfen, verschlossen ist, wobei der Knochenzementteig durch die Austragsöffnung aus der Kartusche auspressbar ist, wenn die Austragsöffnung geöffnet ist, und wobei vorzugsweise der Verschluss für Gase durchlässig und für das Zementpulver undurchlässig ist.

Dadurch kann die Kartusche gut zur Lagerung des Zementpulvers verwendet werden. Der Verschluss kann geöffnet werden. Der Innenraum der Kartusche und das Zementpulver können durch Evakuieren und Spülen des Innenraums der Kartusche mit einem sterilisierenden Gas, wie Ethylenoxid, durch den Verschluss hindurch sterilisiert werden, wenn dieser für Gase durchlässig und für das Zementpulver undurchlässig ist.

Der Verschluss ist vorzugsweise ein für Gase durchlässiger und für das Zementpulver undurchlässiger Filter, insbesondere Porenfilter.

Dabei kann vorgesehen sein, dass der Verschluss an der dem Innenraum der Kartusche zugewandten Rückseite eine Vertiefung aufweist, in der der vorderste Teil des Zementpulvers enthalten ist.

Dadurch wird erreicht, dass der vorderste Teil des Knochenzementteigs, der in der Vertiefung enthalten ist, mit dem Verschluss entfernt werden kann. In diesem Teil ist die Monomerflüssigkeit zuletzt angelangt, wenn sie von der Rückseite aus in das Zementpulver eingepresst wird. Dadurch kann also ein weniger stark durchmischter Teil des Knochenzementteigs mit dem Verschluss entfernt werden.

Der Verschluss bildet vorzugsweise mit dem Austragskolben ein durch axialen Druck auf den Austragskolben in Richtung der Austragsöffnung wirkenden Druck zu öffnendes Verschlusssystem der Kartusche.

Ferner kann vorgesehen sein, dass an der Vorderseite der Kartusche ein Austragsrohr angeordnet, wobei der Knochenzementteig durch das Austragsrohr auspressbar ist.

Hiermit kann die Vorrichtung gut zur Applikation von Knochenzementteig an schwerer zugänglichen Stellen verwendet werden.

Es kann auch vorgesehen sein, dass das Volumen der Zwischenräume zwischen den Zementpartikeln des Zementpulvers im Innenraum der Kartusche im Bereich von 22 Volumenprozent bis 40 Volumenprozent bezogen auf das Gesamtvolumen des Zementpulvers ist. Das Gesamtvolumen des Zementpulvers entspricht bevorzugt dem Volumen des Innenraums der Kartusche, der durch den Austragskolben und durch einen Verschluss in einer Austragsöffnung an der Vorderseite der Kartusche begrenzt ist.

Des Weiteren kann vorgesehen sein, dass der Querschnitt des Innenraums der Kartusche maximal 16 cm² beträgt, bevorzugt maximal 5 cm² beträgt.

Es kann analog auch vorgesehen sein, dass der Innendurchmesser der Kartusche kleiner als 50 mm ist, bevorzugt kleiner als 20 mm ist.

Durch den geringen Innendurchmesser ist der Querschnitt des Innenraums der Kartusche so klein, dass der zähe Knochenzementteig aus der Kartusche mit Hilfe einer manuell angetriebenen Auspressvorrichtung ausgepresst werden kann, auch wenn noch weitere, die Strömung behindernde Leitungen, wie ein Schlauch, ein Applikatorrohr oder ein statischer Mischer in Flussrichtung des Knochenzementteigs vorgesehen sind.

Gemäß einer Weiterbildung kann vorgesehen sein, dass das Volumen der Monomerflüssigkeit in der Vorrichtung, insbesondere der Monomerflüssigkeit in einem Monomerflüssigkeitsbehälter in der Vorrichtung, mindestens so groß ist wie das Volumen der mit Luft gefüllten Zwischenräume zwischen den Zementpulverpartikeln in der Kartusche, bevorzugt mindestens so groß ist wie das Volumen der Flüssigkeitsleitungen zwischen dem Innenraum der Kartusche und dem Innenraum einer Aufnahme, in der die Monomerflüssigkeit enthalten ist, plus das Volumen der mit Luft gefüllten Zwischenräume zwischen den Zementpulverpartikeln in der Kartusche.

Hierdurch kann sichergestellt werden, dass das gesamte Zementpulver von der Monomerflüssigkeit benetzt werden kann und so ein homogener Knochenzementteig erzeugt wird.

Die der vorliegenden Erfindung zugrundeliegenden Aufgaben werden auch gelöst durch ein Verfahren zur Herstellung eines Knochenzementteigs, insbesondere eines pastenförmigen Polymethylmethacrylat-Knochenzementteigs, wobei der Knochenzementteig aus einem Zementpulver und einer Monomerflüssigkeit mit einer Vorrichtung zum Herstellen eines Knochenzementteigs aus einer Monomerflüssigkeit und einem Zementpulver als Ausgangskomponenten des Knochenzementteigs und zum Austragen des gemischten Knochenzementteigs hergestellt wird, die Vorrichtung aufweisend eine Kartusche mit einem zylindrischen Innenraum, wobei der Innenraum der Kartusche an der Vorderseite bis auf eine Austragsöffnung zum Austreiben des Knochenzementteigs verschlossen ist, wobei im Innenraum der Kartusche ein in Richtung der Austragsöffnung drückbarer Austragskolben angeordnet ist und wobei das Zementpulver im Innenraum der Kartusche zwischen der Austragsöffnung und dem Austragskolben angeordnet ist, wobei
an der der Austragsöffnung zugewandten Vorderseite des Austragskolbens ein Verschlussmittel angeordnet ist, das die Austragsöffnung verschließt, wenn der Austragskolben gegen die Vorderseite des Innenraums der Kartusche gedrückt ist, wobei das Verschlussmittel eine weitere Bewegung des Austragskolbens in Richtung der Vorderseite der Kartusche blockiert, so dass der Austragskolben zumindest bereichsweise von der Vorderseite des Innenraums der Kartusche beabstandet ist und ein Totvolumen in dem Innenraum der Kartusche verbleibt, wenn der Austragskolben gegen die Vorderseite des Innenraums der Kartusche gedrückt ist, mit den folgenden nacheinander ablaufenden Schritten:
a) Einsetzen der Vorrichtung in eine Auspressvorrichtung, die Auspressvorrichtung aufweisend eine axial vortreibbare Stange, und Eindrücken der Monomerflüssigkeit in den Innenraum der Kartusche, so dass sich die Monomerflüssigkeit mit dem Zementpulver mischt,
b) der Austragskolben wird mit der Stange in Richtung der Austragsöffnung der Kartusche vorgetrieben, wobei durch die Bewegung des Austragskolbens die Mischung aus dem Zementpulver und der Monomerflüssigkeit aus der Kartusche als Knochenzementteig aus der Vorrichtung ausgetrieben wird, und
c) der Austragskolben auf die Vorderseite der Kartusche trifft, wobei das Verschlussmittel die Austragsöffnung verschließt, eine weitere Bewegung des Austragskolbens in Richtung der Austragsöffnung blockiert wird und eine Restmenge der Mischung in dem Totvolumen im Innenraum der Kartusche verbleibt.

Dabei kann vorgesehen sein, dass in Schritt a) die Monomerflüssigkeit durch zumindest eine für das Zementpulver undurchlässige aber für Gase und die Monomerflüssigkeit durchlässige Verbindung im Austragskolben in die Kartusche gepresst wird, vorzugsweise durch eine Bewegung eines Förderkolbens, der mit der Stange der Auspressvorrichtung angetrieben wird, in die Kartusche gepresst wird.

Hiermit wird erreicht, dass die Flussrichtung der Monomerflüssigkeit die gleiche Richtung hat, wie die Bewegung des Austragskolbens, mit dem der Knochenzementteig aus der Kartusche ausgetrieben wird. Dies hat den Vorteil, dass ein einziger unidirektionaler Antrieb sowohl zum Einpressen der Monomerflüssigkeit als auch zum Ausdrücken des Knochenzementteigs verwendet werden kann. Dadurch sind herkömmliche Auspressvorrichtungen wie manuell angetriebene Kartuschenpistolen für das erfindungsgemäße Verfahren einsetzbar.

Ferner kann vorgesehen sein, dass in Schritt a) zuerst das Einsetzen der Vorrichtung in die Auspressvorrichtung erfolgt, daran anschließend ein Förderkolben, der innerhalb einer an der Rückseite der Kartusche angeordneten Aufnahme an der Rückseite der Aufnahme beweglich gelagert ist, mit der Stange in Richtung der Kartusche vorgetrieben wird, wobei durch die Bewegung des Förderkolbens ein Monomerflüssigkeitsbehälter, in dem die Monomerflüssigkeit enthalten ist, geöffnet wird und die Monomerflüssigkeit aus der Aufnahme in die Kartusche gepresst wird, wobei im Innenraum der Kartusche sich das Zementpulver mit der Monomerflüssigkeit mischt.

Hierdurch ist das Verfahren auch zur vorherigen Lagerung der Ausgangskomponenten geeignet. Dadurch kann das Verfahren jederzeit durch Anwendung eines kompakten Full-Prepacked-Systems angewendet werden.

Dabei kann vorgesehen sein, dass beim Vortreiben des Förderkolbens der zerbrochene oder aufgeschlitzte oder aufgeplatzte Monomerflüssigkeitsbehälter zusammengeschoben wird und gleichzeitig Gas aus der Aufnahme durch eine Verbindung in die Kartusche gedrückt wird und durch das Zementpulver in der Kartusche nach außen gedrückt wird.

Des Weiteren kann vorgesehen sein, dass in Schritt b) durch den auf die Mischung des Zementpulvers mit der Monomerflüssigkeit wirkenden Druck ein Verschluss, insbesondere ein Porenfilter, in einer Austragsöffnung an der Vorderseite der Kartusche bewegt oder hervorgedrückt wird, wobei bevorzugt hierauf der Verschluss aus der Austragsöffnung entfernt wird und besonders bevorzugt danach ein Applikationsrohr an der Vorderseite der Kartusche befestigt wird.

Dadurch kann verhindert werden, dass das in der Kartusche enthaltene Zementpulver aus der Kartusche rieseln kann oder das Pulver von außen verunreinigt wird. Gleichzeitig kann der Inhalt der Kartusche mit einem sterilisierenden Gas, wie Ethylenoxid, sterilisiert werden.

Schließlich kann vorgesehen sein, dass in Schritt c) die Austragsöffnung mit dem Verschlussmittel aufgrund des auf die Rückseite des Austragskolbens wirkenden Drucks von der Stange der Auspressvorrichtung abgedichtet wird.

Hierdurch wird erreicht, dass das Öffnen der Vorrichtung durch den gleichen unidirektionalen Antrieb erfolgen kann, mit dem auch der Knochenzementteig aus der Kartusche ausgetrieben wird. Es ist dann nur ein einziger linearer Antrieb notwendig.

Der Erfindung liegt die überraschende Erkenntnis zugrunde, dass es durch das Verschließen der Austragsöffnung und das Zurückhalten eines kleinen Rests der in der Kartusche entstandenen Mischung des Zementpulvers mit der Monomerflüssigkeit in dem Innenraum der Kartusche gelingt, dass am Ende des Auspressvorgangs kein Knochenzementteig mit einer veränderten Konsistenz ausgetragen wird, da der restliche Knochenzementteig in der Kartusche zurückgehalten wird und die Austragsöffnung verschlossen wird. Bevorzugt wird die Austragsöffnung umso fester verschlossen beziehungsweise umso stärker abgedichtet, je größer die Kraft ist, mit der der Austragskolben weiter vorangetrieben wird. Damit kann vermieden werden, dass der letzte Rest des Knochenzementteigs, der aufgrund eines möglicherweise geänderten Mischungsverhältnisses andere physikalische Eigenschaften aufweisen könnte, noch ausgepresst werden kann.

Die bevorzugte erfindungsgemäße Vorrichtung hat die wesentlichen Vorteile, dass die beiden Ausgangskomponenten des Knochenzementteigs im geschlossenen Zementiersystem gelagert sind und dass die Vermischung der Ausgangskomponenten in der geschlossenen Vorrichtung erfolgt. Das bedeutet, dass die Vorrichtung nicht vom Anwender befüllt werden muss. Es handelt sich dann um ein Full-Prepacked-Zementiersystem. Der medizinische Anwender hat keinerlei Kontakt mit den einzelnen Ausgangskomponenten der Knochenzemente. Die Geruchsbelästigung ist dadurch nur noch minimal. Ein besonderer Vorteil der Vorrichtung besteht auch darin, dass durch das einfache Vorwärtsbewegen einer Stange einer manuell angetriebenen Auspressvorrichtung die Monomerflüssigkeit in das Zementpulver gepresst wird. Dabei wird die zwischen den Zementpulverpartikeln vorhandene Luft durch die Monomerflüssigkeit verdrängt. Es entsteht ein homogener Knochenzementteig, ohne dass ein manuelles Mischen mit Mischstäben mit Mischflügeln notwendig ist. Das bedeutet, dass das fehlerbehaftete manuelle Mischen nicht mehr notwendig ist. Die Bedienung der Vorrichtung ist maximal vereinfacht. Es handelt sich um ein Ready-to-use-System.

Die Vorteile erfindungsgemäßer Vorrichtungen und Verfahren beruhen grundsätzlich darauf, dass die an sich bekannte lineare Vorwärtsbewegung von Stangen von manuell betriebenen Auspressvorrichtungen so genutzt werden, dass durch kontinuierliche Einwirkung der Kraft der linearen Vorwärtsbewegung der Stange zuerst ein Monomerflüssigkeitsbehälter geöffnet wird, der Monomerflüssigkeitsbehälter anschließend zusammengepresst wird, wodurch die Monomerflüssigkeit aus dem Monomerflüssigkeitsbehälter austritt und in verdichtetes Zementpulver eingepresst wird, wobei die zwischen den Zementpulverpartikeln vorhandene Luft durch die eingepresste Monomerflüssigkeit verdrängt wird und nach Benetzung der Zementpulverpartikel durch die Monomerflüssigkeit ein Knochenzementteig entsteht. Voraussetzung dafür ist die Verwendung eines Zementpulvers, das so eingestellt ist, dass es sehr gut von der Monomerflüssigkeit benetzt wird und diese durch Kapillarwirkung einsaugen kann.

Die Vorrichtung kann als hygienisches Wegwerfprodukt verwendet werden, da sie sehr weitgehend aus Kunststoff gefertigt werden kann und weil alle Teile einschließlich der Innenräume und des Zementpulvers mit Hilfe von Ethylenoxid sterilisierbar sind.

Die erfindungsgemäße Vorrichtung ist dadurch charakterisiert, dass der Austragskolben an der zum Kartuschenkopf zeigenden Stirnseite einen Zapfen besitzt, das an dem zum Kartuschenkopf zeigenden Ende konusförmig ausgebildet ist, wobei der Durchmesser der Basis des Konus einen größeren Durchmesser hat als der Innendurchmesser der rohrförmigen Austragsöffnung beziehungsweise des Austragsrohrs. Dabei ist vorzugsweise der Zapfen so angeordnet, dass bei axialer Bewegung des Austragskolbens in Richtung des Kartuschenkopfs zu mindestens teilweise in das Austragsrohr eintaucht oder abdeckt beziehungsweise abdichtet und dieses flüssigkeitsundurchlässig verschließt.

Die Erfindung basiert auch auf der Idee, dass der Zapfen des Austragskolbens am Ende des Auspressvorgangs des Knochenzementteigs in Folge der axialen Bewegung des Austragskolbens in Richtung Kartuschenkopf in die Austragsöffnung mit seinem Konus eintaucht oder auf einen Stutzen an der Austragsöffnung aufgesteckt wird. Bei weiterer axialer Bewegung des Austragskolbens in Richtung des Kartuschenkopfs wird der Konus in die röhrenförmige Austragsöffnung immer stärker eingepresst oder auf den Stutzen immer stärker aufgepresst. Eine Bewegung des Austragskolbens in Richtung Kartuschenkopf wird dadurch verhindert und gleichzeitig wird die Austragsöffnung flüssigkeitsdicht verschlossen und abgedichtet. Bei einer möglichen Nachkompression des geborstenen Monomerflüssigkeitsbehälters kann dadurch keine Monomerflüssigkeit durch die Austragsöffnung austreten. Das bedeutet, die Kartusche verschließt sich nach erfolgtem Austrag des Hauptteils des Knochenzementteigs bei weiterer Vorwärtsbewegung der Stange der Auspressvorrichtung selbst.

Eine beispielhafte Vorrichtung zum Lagern, Vermischen und Austragen von Polymethylmethacrylat-Knochenzement kann beispielsweise aufweisen:
a) einen hohlzylinderförmigen Behälter mit einem am Kartuschenende angeordneten Verbindungselement zur Verbindung mit einer Auspressvorrichtung,
b) einen Kartuschenkopf, der den hohlzylinderförmigen Behälter an der Vorderseite abschließt, wobei eine Durchführung zur Aufnahme des Austragsrohrs als Austragsöffnung im Kartuschenkopf angeordnet ist, und wobei mindestens eine Durchführung die Außenseite des Kartuschenkopfs mit der Innenseite des Kartuschenkopfs gasdurchlässig verbindet,
c) ein Austragsrohr,
d) einen axial im Kartuschenkopf beweglichen Verschluss, der gasdurchlässig aber für Pulverpartikel undurchlässig ist, wobei der Verschluss eine von der Unterseite zur Oberseite gehende Durchführung besitzt, die an der Oberseite mit dem Austragsrohr flüssigkeitsdurchlässig verbunden ist,
e) einen Förderkolben, der in dem Behälter axial beweglich angeordnet ist, der den Kartuschenboden flüssigkeitsundurchlässig verschließt,
f) einen Austragskolben, der in dem Behälter axial beweglich zwischen dem Verschluss und dem Förderkolben angeordnet ist, wobei der Austragskolben mindestens eine flüssigkeitsdurchlässige und für Pulverpartikel undurchlässige Verbindung zwischen den beiden Stirnseiten besitzt,
g) mindestens einen Monomerflüssigkeitsbehälter, der zwischen dem Austragskolben und dem Förderkolben in dem Behälter angeordnet ist,
h) einen Innenraum (den Innenraum der Kartusche), in dem das Zementpulver angeordnet ist, wobei der Innenraum durch die Innenwand des Behälters, den Verschluss und den Austragskolben begrenzt ist.

Der Behälter umfasst dabei die Kartusche als den vorderen Teil des Behälters, in dem das Zementpulver angeordnet ist, und eine Aufnahme als den hinteren Teil des Behälters, in dem der Monomerflüssigkeitsbehälter angeordnet ist.

Ein Verfahren kann beispielsweise mit der beispielhaften Vorrichtung zur Vermischung des Zementpulvers mit der Monomerflüssigkeit unter Bildung von Knochenzementteig mit den aufeinanderfolgenden Schritten umgesetzt werden:
a) Verbinden der Auspressvorrichtung mit dem Verbindungselement des Behälters,
b) Vortreiben der Stange der Auspressvorrichtung,
c) Verschieben des Förderkolbens in Richtung des Kartuschenkopfs,
d) Komprimierung des mindestens einen Monomerflüssigkeitsbehälters zwischen dem Austragskolben und dem Förderkolben,
e) Bersten oder Reißen des Monomerflüssigkeitsbehälters,
f) Zusammenschieben des geborstenen oder gerissenen Monomerflüssigkeitsbehälters und Auspressen der Luft aus dem Innenraum der Aufnahme und der Monomerflüssigkeit mit dem Förderkolben durch die mindestens eine Verbindung des Austragskolbens in das Zementpulver in den Innenraum der Kartusche,
g) Ausbreitung der Monomerflüssigkeit in dem Zementpulver unter gleichzeitiger Verdrängung der Luft aus den Zwischenräumen der Zementpulverpartikel,
h) Benetzung der Zementpulverpartikel mit der Monomerflüssigkeit,
i) Entweichen der Luft aus dem Zementpulver durch den gasdurchlässigen Verschluss,
j) Anquellen der Zementpulverpartikel durch die Monomerflüssigkeit und Auslösung der radikalischen Polymerisation der Monomerflüssigkeit durch Reaktion des Beschleunigers mit dem Initiator,
k) Bildung des Knochenzementteigs aus dem Zementpulver und der Monomerflüssigkeit,
l) Öffnung des Verschlusses in der Austragsöffnung durch axiale Druckbeaufschlagung durch den axial in Richtung Kartuschenkopf gepressten Knochenzementteig,
m) Auspressen des Knochenzementteigs durch die Austragsöffnung infolge der Vorwärtsbewegung des Austragskolbens und des Förderkolbens, und
n) Axiale Bewegung des Austragskolbens in Richtung des Kartuschenkopfs, bis der Zapfen in die Austragsöffnung eintaucht und diese verschließt oder die Kappe auf die Austragsöffnung gesteckt wird diese verschließt oder das Verschlussmittel die Austragsöffnung abdichtet und verschließt.

Eine beispielhafte Variante des Verfahrens ist durch die folgenden Schritte nach dem Schritt k) des zuvor beschriebenen Verfahrens charakterisiert:
k1) Auspressen des Verschlusses aus der Austragsöffnung, und
k2) Herausfallen des Verschlusses aus der Austragsöffnung oder einem Austragsrohr.

Im Folgenden werden weitere Ausführungsbeispiele der Erfindung anhand von neun schematisch dargestellten Figuren erläutert, ohne jedoch dabei die Erfindung zu beschränken. Dabei zeigt:
Figur 1: eine schematische Querschnittansicht einer beispielhaften erfindungsgemäßen Vorrichtung zum Lagern und Mischen einer Monomerflüssigkeit und eines Zementpulvers, die in eine Auspressvorrichtung eingesetzt ist;
Figur 2: eine schematische Seitenansicht der Vorrichtung nach Figur 1, die nicht in die Auspressvorrichtung eingesetzt ist;
Figur 3: fünf schematische Querschnittansichten der Vorrichtung nach den Figuren 1 und 2 mit einer angeschlossenen Auspressvorrichtung übereinander zur Verdeutlichung des Ablaufs eines erfindungsgemäßen Verfahrens;
Figur 4: eine schematische Querschnittansicht als Ausschnittvergrößerung durch den vorderen Teil der erfindungsgemäßen Vorrichtung nach den Figuren 1 bis 3 mit vorgeschobenem Porenfilter;
Figur 5: drei schematische perspektivische Ansichten auf erfindungsgemäße Vorrichtungen nach den Figuren 1 bis 4 mit Applikationsrohr, ohne Aufsatz und mit Kappe am Austragsrohr;
Figur 6: eine schematische Querschnittansicht als Ausschnittvergrößerung der Vorrichtung im Ausgangszustand nach der ersten Abbildung von oben von Figur 3, wobei die Vorrichtung in die Auspressvorrichtung eingesetzt ist;
Figur 7: eine schematische Querschnittansicht als Ausschnittvergrößerung der Vorrichtung nach der zweiten Abbildung von oben von Figur 3 beim Einpressen der Monomerflüssigkeit;
Figur 8: eine schematische Querschnittansicht als Ausschnittvergrößerung der Vorrichtung nach der vierten Abbildung von oben von Figur 3 beim Austragen des Knochenzementteigs; und
Figur 9: eine schematische Querschnittansicht als Ausschnittvergrößerung der Vorrichtung nach der letzten Abbildung von oben von Figur 3 mit verschlossenem Austragsrohr beziehungsweise verschlossener Austragsöffnung.

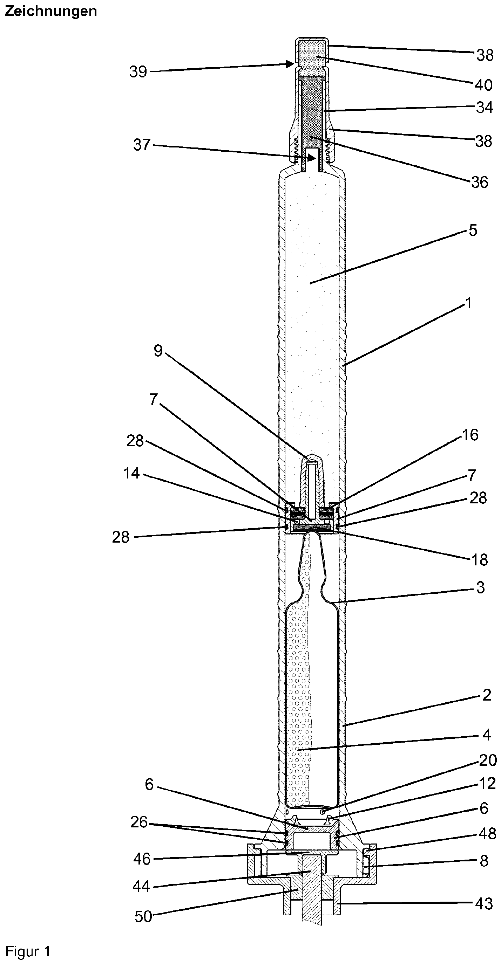

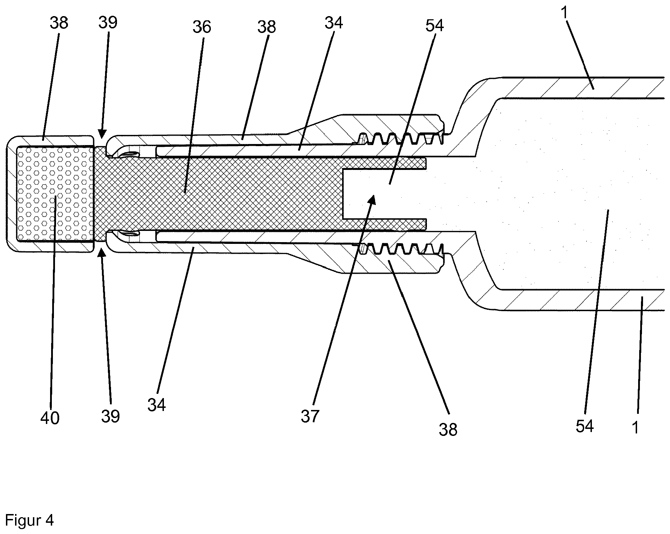

In den Figuren 1 bis 9 sind Abbildungen einer erfindungsgemäßen Vorrichtung gezeigt. Die Figuren 1 bis 3 und 5 zeigen verschiedene schematische Gesamtansichten der beispielhaften erfindungsgemäßen Vorrichtung. Die Figuren 4 und 6 bis 9 zeigen schematische Querschnittansichten als Detailansichten in Form von Ausschnittvergrößerungen durch verschiedene Bereiche der erfindungsgemäßen Vorrichtung.

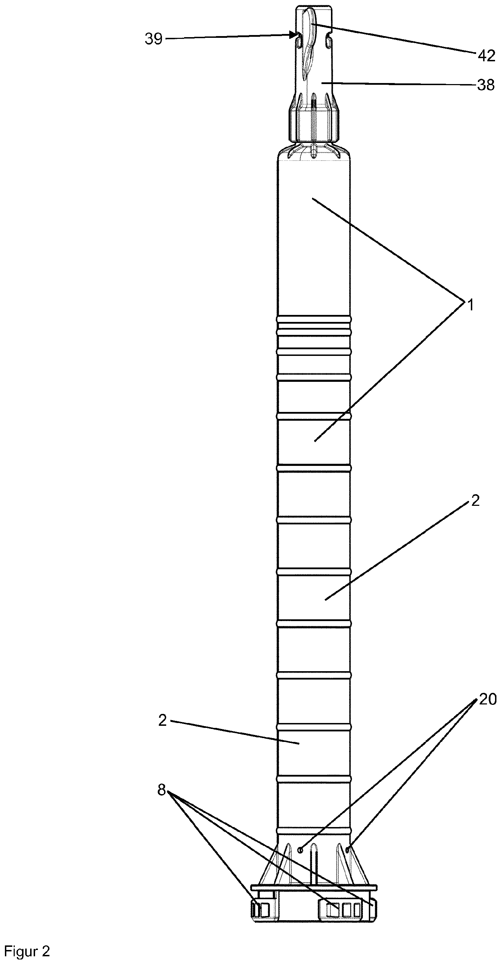

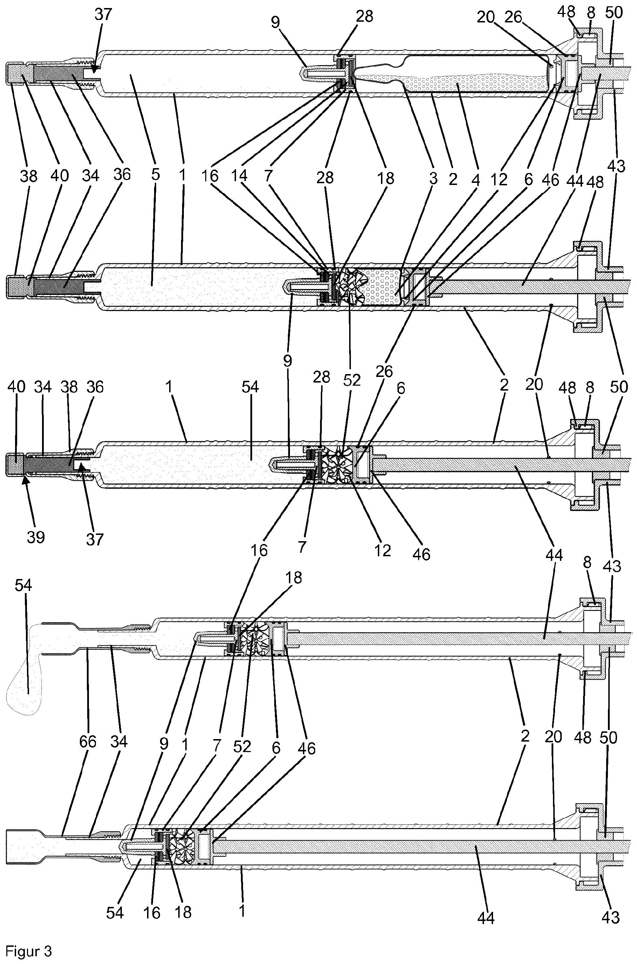

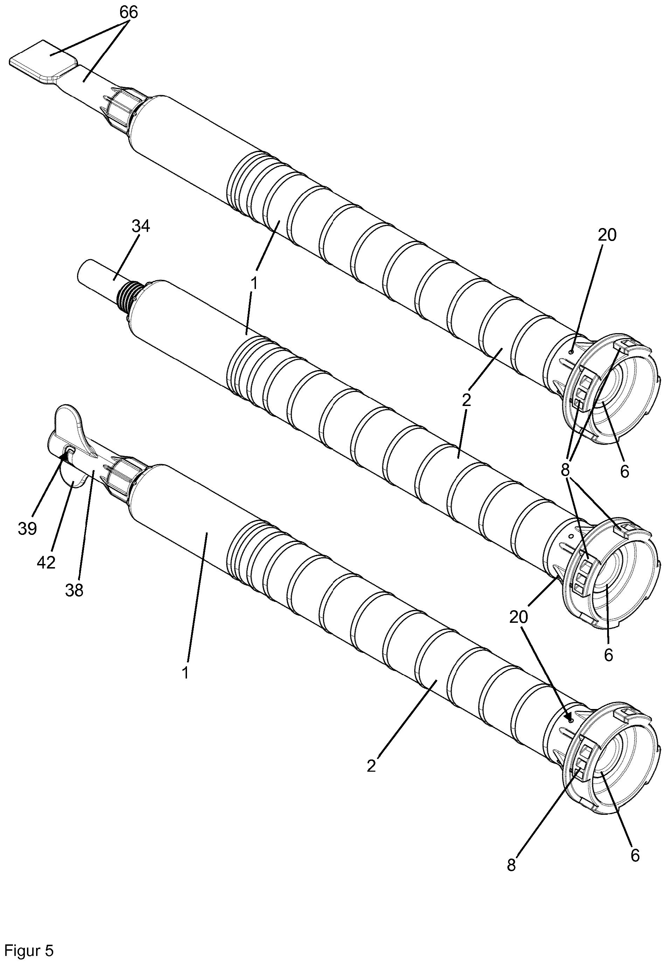

Die erfindungsgemäße Vorrichtung besteht im Wesentlichen aus einem röhrenförmigen Behälter aus Kunststoff, der als vorderen Teil (in den Figuren 1 und 2 oben, in den Figuren 3, 4, 6 bis 9 links und in Figur 5 links oben) eine Kartusche 1 mit einem zylindrischen Innenraum bildet und der als hinteren Teil eine Aufnahme 2 für eine Glasampulle 3 (oder Kunststoffampulle 3) als Monomerflüssigkeitsbehälter bildet. Die Rückseite der Vorrichtung ist in den Figuren 1 und 2 unten, in den Abbildungen der Figur 3 rechts und in Figur 5 unten rechts sowie in den Figuren 4 und 6 bis 9 gezeigt. Die Röhrenform des Behälters ist besonders gut in den Querschnittansichten der Figuren 1 und 3 zu erkennen. Sowohl der Innenraum der Kartusche 1 als auch der Innenraum der Aufnahme 2 sind zylindrisch mit kreisförmiger Grundfläche. Dabei sind die Durchmesser des Innenraums der Kartusche 1 und der Durchmesser des Innenraums der Aufnahme 2 gleich groß. Der Behälter mit der Aufnahme 2 und der Kartusche 1 wird vorzugsweise mit Hilfe einer Spritzgusstechnik aus Kunststoff hergestellt. Die Aufnahme 2 weist also einen zylindrischen Innenraum auf, in den die Glasampulle 3 eingesteckt ist. In der Glasampulle 3 befindet sich die Monomerflüssigkeit 4. In den Innenraum der Kartusche 1 ist ein Zementpulver 5 eingefüllt oder vorzugsweise eingepresst. Die Monomerflüssigkeit 4 und das Zementpulver 5 bilden die Ausgangskomponenten für einen PMMA-Knochenzement, der mit der Vorrichtung hergestellt werden kann. Aufgrund der Glasampulle 3 kann die Monomerflüssigkeit 4 sehr lange in der Aufnahme 2 und dadurch in der Vorrichtung gelagert werden. Das Zementpulver 5 kann ebenfalls über längere Zeiträume in der Vorrichtung gelagert werden. Die Vorrichtung ist damit zum Lagern der Monomerflüssigkeit 4 und des Zementpulvers 5 als Ausgangskomponenten eines Knochenzementteigs des PMMA-Knochenzements geeignet. Die Vorrichtung ist aber auch zum Mischen des Knochenzementteigs aus den Ausgangskomponenten und zum Austragen des gemischten Knochenzementteigs geeignet und vorgesehen.

In der Aufnahme 2 ist ein im zylindrischen Innenraum der Aufnahme 2 in Längsrichtung beweglicher Förderkolben 6 aus Kunststoff angeordnet. Der Förderkolben 6 ist im Bereich der Rückseite der Aufnahme 2 angeordnet. Die Glasampulle 3 kann mit dem Förderkolben 6 in der Aufnahme 2 zusammengedrückt und dabei zersplittert werden, indem der Förderkolben 6 in Richtung der Vorderseite, also in Richtung der Kartusche 1 gedrückt wird. Der Förderkolben 6 weist an der Vorderseite Abstreifer auf, mit denen Splitter der Glasampulle 3 von der Innenwand der Aufnahme 2 abgestreift werden. Die Abstreifer liegen hierzu seitlich an der Innenwand des Innenraums der Aufnahme an.

In dem Innenraum der Kartusche 1 ist in dessen Rückseite (in den Figuren 1 und 2 Richtung unten, in den Figuren 3 und 4 bis 9 Richtung rechts) ein Austragskolben 7 aus Kunststoff angeordnet. An der Rückseite der Aufnahme 2 ist ein Befestigungsmittel 8 vorgesehen, mit dem die Aufnahme 2 an einer Auspressvorrichtung 43 (in Figur 2 nicht zu sehen, siehe aber Figuren 1, 3 und 6) verbunden werden kann. Das Befestigungsmittel 8 ist vorzugsweise zur Bildung eines Bajonettverschlusses 8 geeignet und vorgesehen. Dadurch kann der Förderkolben 6, der von der Rückseite der Aufnahme 2 aus frei zugänglich ist, mit der Auspressvorrichtung 43 in Richtung der Vorderseite der Kartusche 1 vorgetrieben werden.

Der Austragskolben 7 weist an seiner Vorderseite einen Zapfen 9 als Verschlussmittel zum Verschließen einer Austragsöffnung an der Vorderseite des Innenraums der Kartusche 1 auf. Der Zapfen 9 ist rotationssymmetrisch und weist einen steilen und einen flachen konischen Bereich auf. Der steile konische Bereich des Zapfens 9 wird zum Abdichten der Austragsöffnung an der Vorderseite der Kartusche 1 verwendet.

An der Vorderseite des Förderkolbens 6 sind Keile 12 angeordnet, die zum Spalten oder Brechen der Glasampulle 3 bei Vortreiben des Förderkolbens 6 vorgesehen sind.

Die Kartusche 1 und die Aufnahme 2 sind einteilig als gemeinsames Kunststoffteil ausgeführt. Die Aufnahme 2 und die Kartusche 1 sind für die Monomerflüssigkeit 4 flüssigkeitsdurchlässig miteinander über eine Verbindung 14 im Austragskolben 7 verbunden. Die Verbindung 14 durch den Austragskolben 7 mündet durch einen für das Zementpulver 5 undurchlässigen aber für die Monomerflüssigkeit 4 durchlässigen Porenfilter 16 in den Innenraum der Kartusche 1.

In der Einmündung zur Verbindung 14 ist in dem Austragskolben 7 ein Filter 18 angeordnet, mit dem die Splitter der Glasampulle 3 zurückgehalten werden können. Statt dem Filter 18 oder zusätzlich zum Filter 18 kann auch ein Sieb vorgesehen sein.

Mehrere Belüftungsöffnungen 20 sind in der Wandung der Aufnahme 2 vorgesehen, durch die der Innenraum der Aufnahme 2 mit Hilfe eines sterilisierenden Gases wie Ethylenoxid sterilisiert werden kann. Die Belüftungsöffnungen 20 sind unmittelbar benachbart zum Förderkolben 6 angeordnet, so dass der Förderkolben 6 sich unmittelbar vor die Belüftungsöffnungen 20 schiebt und somit die Belüftungsöffnungen 20 unmittelbar verschließt, wenn der Förderkolben 6 in Richtung der Kartusche 1 vorgetrieben wird. Dadurch wird verhindert, dass Monomerflüssigkeit 4 durch die Belüftungsöffnungen 20 austreten kann, wenn die Glasampulle 3 in der Aufnahme 2 geöffnet wurde.

Der zylindrische Förderkolben 6 hat einen zur Zylindergeometrie des Innenraums der Aufnahme 2 passenden Außenumfang und ist über zwei umlaufende Dichtungen 26 gegen die Innenwand der Aufnahme 2 flüssigkeitsdicht abgedichtet. Ebenso ist der Austragskolben 7 über zwei umlaufende Dichtungen 28 gegen die Innenwand der Kartusche 1 flüssigkeitsdicht abgedichtet. Diese Dichtungen 26, 28 dienen dazu, einen Austritt von Monomerflüssigkeit 4 oder von Knochenzement zu verhindern, um eine Kontamination der Umgebung (des OP-Saals und des Anwenders) zu verhindern. Die Dichtungen 26, 28 können hierzu aus Gummi bestehen.

Der Innenraum der Kartusche 1 mündet an der Vorderseite in ein Austragsrohr 34, das die Austragsöffnung der Kartusche 1 begrenzt. Das Austragsrohr 34 weist an seiner Basis ein Außengewinde auf. Im Inneren der Austragsrohrs 34 ist ein Porenfilter 36 als Verschluss für die Kartusche 1 angeordnet. Der Porenfilter 36 ist für das Zementpulver 5 undurchlässig aber für Gase durchlässig. In der Rückseite des Porenfilters 36 ist eine Vertiefung 37 vorgesehen. Das Zementpulver 5 ist auch in der Vertiefung 37 enthalten. An dem Außengewinde des Austragsrohrs 34 ist eine Kappe 38 befestigt, wobei der vordere Teil der Kappe 38 mit einem Styropor oder Schaumstoff 40 gefüllt ist. An der Kappe 38 sind zwei Flügel 42 vorgesehen, so dass die Kappe 38 nach Art einer Flügelschraube bequem von dem Austragsrohr 34 abgeschraubt werden kann. Die Kappe 38 weist seitliche Öffnungen 39 auf. Durch diesen Aufbau kann das Innere der Kartusche 1 und das Zementpulver 5 mit Hilfe von Ethylenoxid sterilisiert werden, da die Öffnungen 39 in der Kappe 38, das Styropor oder der Schaumstoff 40, der Porenfilter 36 und die Zwischenräume zwischen den Pulverpartikeln des Zementpulvers 5 luftdurchlässig sind. Gleichzeitig kann Luft aus der Aufnahme 2 durch das Zementpulver 5, den Porenfilter 36, das Styropor oder der Schaumstoff 40 und die Öffnungen 39 in der Kappe 38 herausgepresst werden, wenn der Förderkolben 6 in Richtung der Aufnahme 1 gepresst wird. Die Kappe 38 bildet zusammen mit dem Styropor oder Schaumstoff 40 und mit dem Porenfilter 36 einen Verschluss für die Austragsöffnung der Kartusche 1 beziehungsweise für das Austragsrohr 34.

Das Zementpulver 5 ist in der Kartusche 1 eingeschlossen, da alle Öffnungen 39 und Verbindungen 14 mit Hilfe der Porenfilter 16, 36 für das Zementpulver 5 undurchlässig verschlossen sind. Der Inhalt der Kartusche 1 kann dabei durch Evakuieren und Spülen mit Ethylenoxid sterilisiert werden. Dadurch ist die Vorrichtung auch zum langfristigen Lagern des Zementpulvers 5 geeignet.

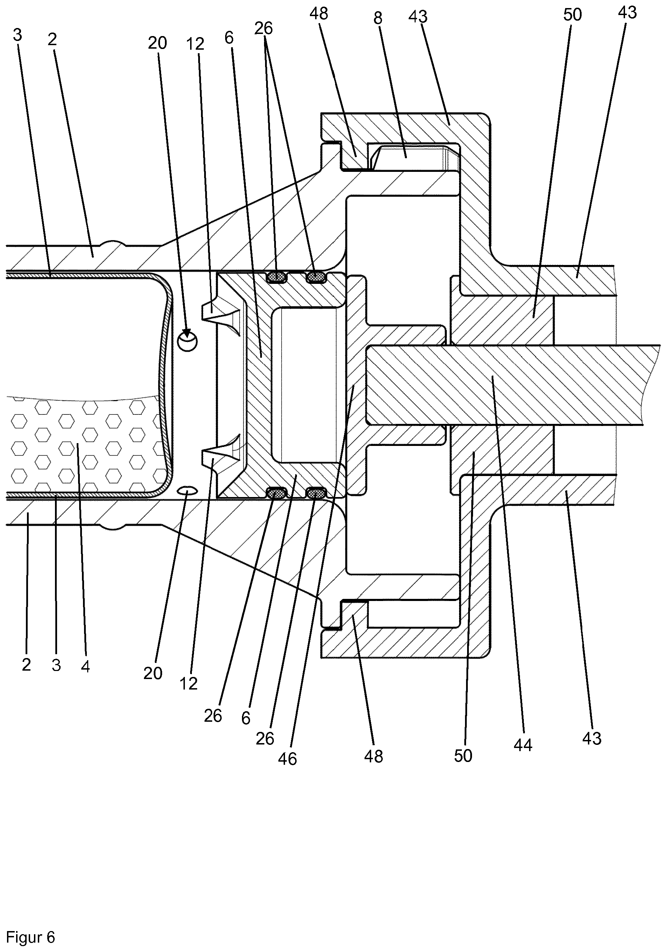

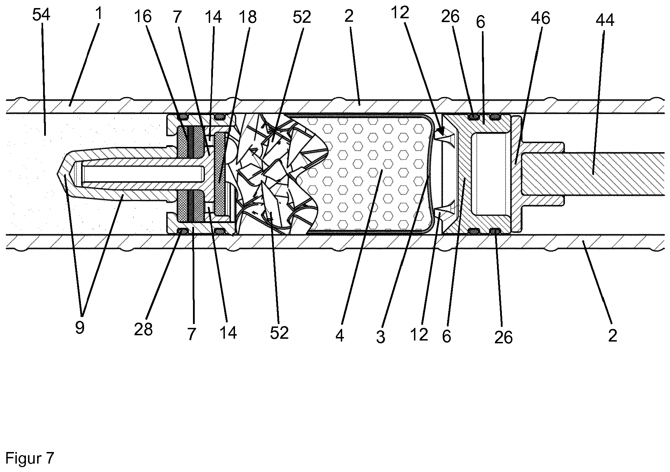

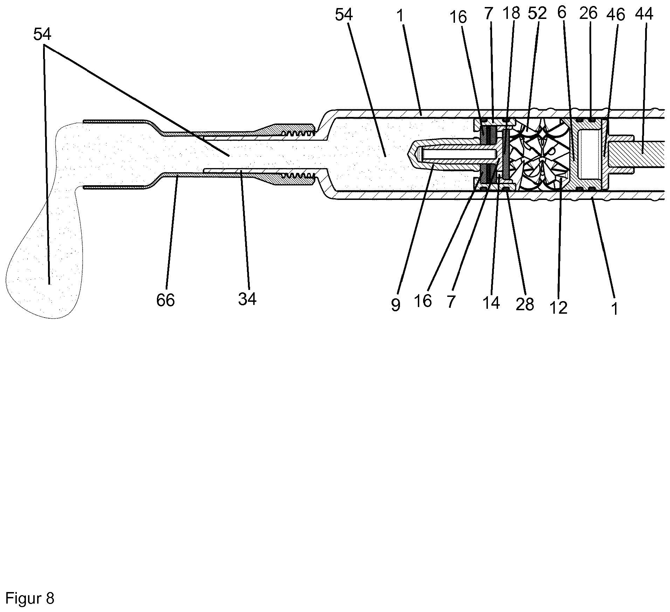

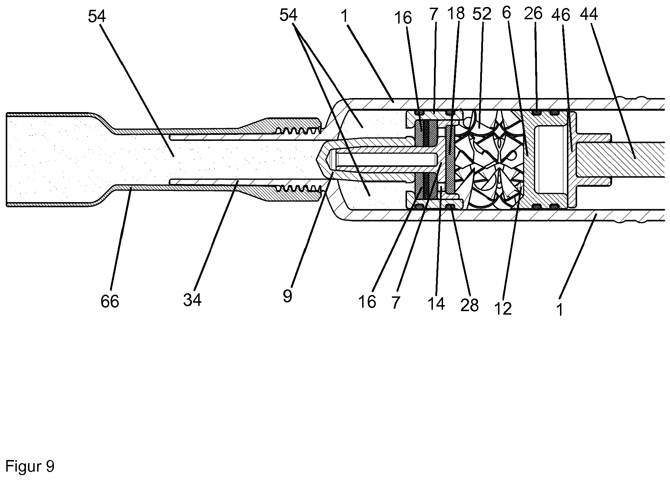

Figur 3 zeigt fünf schematische Querschnittansichten der erfindungsgemäßen Vorrichtung nach den Figuren 1 und 2 übereinander zur Verdeutlichung des Ablaufs eines erfindungsgemäßen Verfahrens. Dazu zeigt Figur 4 eine Ausschnittvergrößerung der dritten Abbildung der Figur 3, Figur 6 eine Ausschnittvergrößerung der ersten Abbildung von oben der Figur 3, Figur 7 eine Ausschnittvergrößerung der zweiten Abbildung von oben der Figur 3, Figur 8 eine Ausschnittvergrößerung der vierten Abbildung von oben der Figur 3 und Figur 9 eine Ausschnittvergrößerung der untersten Abbildung der Figur 3.

Zu Beginn des Verfahrens liegt die Vorrichtung im Ausgangszustand vor, so wie sie auch in Figur 1 gezeigt ist. In diesem Zustand wird die Vorrichtung in eine erfindungsgemäße Auspressvorrichtung 43 eingesetzt, die weitgehend einer herkömmlichen Kartuschenpistole entspricht. Diese Situation ist in Figur 1 sowie der obersten Abbildung von Figur 3 und in Figur 6 gezeigt. Die Auspressvorrichtung 43 umfasst eine linear vortreibbare Stange 44. Von der Auspressvorrichtung 43 ist nur der vordere Teil dargestellt. Die Auspressvorrichtung 43 umfasst auch einen Griff und einen Kipphebel (in den Abbildungen nicht zu sehen) zum manuellen Antreiben der Stange 44 der Auspressvorrichtung 43, wie bei herkömmlichen manuell angetriebenen Auspressvorrichtungen auch. Die Vorrichtung wird mit dem Befestigungsmittel 8 an der Auspressvorrichtung 43 befestigt (siehe oberste Abbildung in Figur 3 sowie im Detail in Figur 6). An der Spitze der Stange 44 ist ein flacher Teller 46 zum Antreiben des Förderkolbens 6 vorgesehen. Die Stange 44 drückt mit dem Teller 46 auf den Förderkolben 6, wenn die Stange 44 von der Auspressvorrichtung 43 in die Aufnahme 2 hineingedrückt wird. Die Auspressvorrichtung 43 ist hierzu über ein Gegenbefestigungsmittel 48 an die Rückseite der Aufnahme 2 angeschlossen, so dass der Teller 46 beim Vortreiben der Stange 44 auf den Förderkolben 6 drückt und diesen in Richtung der Kartusche 1 vortreibt.

Hierzu ist die Stange 44 gegen ein Lager 50 und darüber gegen das Gegenbefestigungsmittel 48 und damit gegen die Aufnahme 2 linear beweglich gelagert.

Die Auspressvorrichtung 43 wird bedient und dabei die Stange 44 und mit der Stange 44 der Förderkolben 6 in Richtung der Kartusche 1 vorgetrieben. Da die Glasampulle 3 an der Vorderseite an dem Austragskolben 7 anliegt, verkleinert sich der Innenraum der Aufnahme 2 und die Glasampulle 3 zerbricht. Die Monomerflüssigkeit 4 tritt aus der Glasampulle 3 in den Innenraum der Aufnahme 2 aus. Der Austragskolben 7 kann nicht oder nicht weit von der Glasampulle 3 in Richtung des Porenfilters 36 geschoben werden, wenn das Zementpulver 5 trocken ist, also nicht von der Monomerflüssigkeit 4 benetzt ist, da das trockene Zementpulver 5 nicht fließfähig ist und eine Bewegung des Austragskolbens 7 blockiert. Diese Situation ist in Figur 3, zweite Abbildung von oben, sowie in der vergrößerten Ausschnittansicht in Figur 7 gezeigt. Überstehende Luft aus der Aufnahme 2 wird durch den Filter 18, die Verbindung 14, den Porenfilter 16, durch die Zwischenräume zwischen den Partikeln des Zementpulvers 5, durch den Porenfilter 36, durch den Schaumstoff 40 und aus den Öffnungen 39 in der Kappe 38 aus der Vorrichtung herausgedrückt.

Von der Glasampulle 3 bleiben schließlich nur kleine Splitter 52 zurück, die von dem Filter 18 zurückgehalten werden und in dem röhrenförmigen Behälter verbleiben. Die Monomerflüssigkeit 4 wird durch den Filter 18, die Verbindung 14 und den Porenfilter 16 in das Zementpulver 5 gepresst und beginnt dort mit dem Zementpulver 5 zu reagieren, so dass sich aus dem Gemisch 54 der Knochenzementteig 54 bildet. Die Menge der Monomerflüssigkeit 4 ist so gewählt, dass das Zementpulver 5 bis in die vorderste Spitze der Kartusche 1, das heißt bis in die Vertiefung 37 im Porenfilter 36 mit der Monomerflüssigkeit 4 benetzt wird. Diese Situation ist in Figur 3, dritte Abbildung von oben gezeigt. Der Porenfilter 36 wird, sobald das Gemisch 54 entstanden ist, von dem auf das Gemisch 54 aufgrund des Drucks auf den Austragskolben 7 wirkenden Druck nach vorne getrieben und komprimiert den Schaumstoff 40. Wenn der Porenfilter 36 nun nach vorne rutscht, so wird er von außen für den Anwender durch die Öffnung 39 in der Kappe 38 sichtbar. Diese Situation ist in Figur 4 im Detail gezeigt. Hierzu hat der Porenfilter 36 vorzugsweise eine andere Farbe und/oder Helligkeit als der Schaumstoff 40. Beispielsweise kann der Schaumstoff 40 weiß sein und der Porenfilter 36 rot.

In diesem Zustand wird die Kappe 38 mit dem Porenfilter 36 und dem Schaumstoff 40 abgeschraubt und stattdessen eine verlängerte Austragsöffnung in Form eines Applikatorrohrs 66 auf das Austragsrohr 34 aufgeschraubt (siehe auch Figur 5). Beim Abschrauben der Kappe 38 wird der vorderste Teil des Gemischs 54 beziehungsweise des Knochenzementteigs 54, der sich in der Vertiefung 37 des Porenfilters 38 befindet, mit der Kappe 38 und dem Porenfilter 36 entfernt. Dadurch wird ein potenziell schlechter gemischter Teil des Knochenzementteigs 54 entfernt und somit eine größere Homogenität des verfügbaren Knochenzementteigs 54 erreicht.

Durch weiteres Vortreiben der Stange 44 wird der Förderkolben 6, die Scherben 52 und der davor angeordnete Austragskolben 7 angetrieben. Über das Applikatorrohr 66 wird dann der Knochenzementteig 54 aus der Kartusche 1 ausgetragen. Dazu wird der Austragskolben 7 mit der Stange 44 in Richtung des Austragsrohrs 34 vorgetrieben (siehe hierzu auch die vierte Abbildung von oben der Figur 3 sowie die Detailansicht nach Figur 8). Der Knochenzementteig 54 aus dem Inneren der Kartusche 1 wird durch das Austragsrohr 34 und das Applikatorrohr 66 ausgetrieben und kann dort appliziert werden oder theoretisch zur weiteren Verarbeitung verwendet werden.

Schließlich trifft der Zapfen 9 auf die Austragsöffnung der Kartusche 1. Der konische Zapfen 9 wird dabei zunächst in das Austragsrohrs 34 eingeführt. Da die Basis das konischen Zapfens 9 einen größeren Außendurchmesser aufweist als der Innendurchmesser des zylindrischen Austragsrohrs 34 beziehungsweise der kreisrunden Austragsöffnung, wird der konische Bereich des Zapfens 9 mit der Kante, die die Verbindung der Austragsöffnung mit dem Innenraum der Kartusche 1 bildet, schließen. Die kreisförmige Kante der Austragsöffnung beziehungsweise des Austragsrohrs 34 wird dabei in die konische Mantelfläche des Zapfens 9 gedrückt. Diese Situation ist in der untersten Abbildung von Figur 3 gezeigt sowie in Figur 9 als Ausschnittvergrößerung. Je stärker der Austragskolben 7 nach vorne gedrückt wird, desto dichter wird der Innenraum der Kartusche 1 schließen.

Da am Ende des Auspressvorgangs der Austragskolben 7 blockiert wird, kann es dazu kommen, dass die Scherben und Splitter 52 der Glasampulle 3 durch den auf die Scherben und Splitter 52 wirkenden wachsenden Druck noch weiter komprimiert werden und dabei noch weitere Reste der Monomerflüssigkeit 4 aus dem Zwischenraum zwischen dem Austragskolben 7 und dem Förderkolben 6 in den vorderen Teil der Kartusche 1 gedrückt werden. Dadurch kann es zu einer Veränderung der Zusammensetzung des Knochenzementteigs 54 kommen, da der Anteil an flüssiger Monomerflüssigkeit 4 in der Mischung 54 erhöht wird. Wenn der Knochenzementteig 54 schon sehr weitgehend reagiert hat kann es auch sein, dass sich die Monomerflüssigkeit 4 einen Weg an dem Knochenzementteig 54 vorbei bahnt. Durch die Länge des Zapfens 9 ist gewährleistet, dass die Vorderseite des Austragskolbens 7 von der Vorderseite des Innenraums der Kartusche 1 beabstandet ist, wenn der Austragskolben 7 so weit nach vorne gedrückt es, wie es mit einer manuell angetriebenen Auspressvorrichtung 43 möglich ist. Dadurch entsteht im Innenraum der Kartusche 1 ein Totvolumen, das nicht aus der Kartusche 1 durch die Austragsöffnung und das Austragsrohr 34 ausgetrieben werden kann.

In diesem Totvolumen befindet sich nun der Teil des Knochenzementteigs 54, der gegebenenfalls einen zu großen Anteil an Monomerflüssigkeit 4 enthält. Auch wenn nachfolgend immer weiter gepresst wird, so wird nur noch die Dichtwirkung des Zapfens 9 weiter erhöht. Durch diesen Aufbau wird sichergestellt, dass kein Knochenzementteig 54 mit einer sich ändernden Konsistenz aufgrund sich ändernder Zusammensetzung mit der Vorrichtung appliziert werden kann.

Ferner kann natürlich anstatt des Verschlussmittels auch das Austragsrohr beziehungsweise die Austragsöffnung eine konische Fläche aufweisen, die mit einer Kante oder einer ebenfalls konischen Fläche des Verschlussmittels abdichtet. Anstatt konische Flächen können auch abgerundete Oberflächen zur Abdichtung verwendet werden. So kann beispielsweise anstatt des Zapfens 9 mit konischer Fläche auch ein Zapfen mit einer eiförmigen oder ellipsoiden Oberfläche zum Abdichten verwendet werden. Wichtig ist bei all diesen Ausführungen des Verschlussmittels, dass eine Dichtwirkung erzielt wird. Hierzu ist es ausreichend, wenn ein vollumfänglicher Schluss gegen die Außenabgrenzung der Austragsöffnung erreicht wird. Vorzugsweise soll dabei eine weitere Bewegung des Verschlussmittels in oder auf die Austragsöffnung zu einer Verstärkung der Dichtwirkung führen.

Die Öffnungen 39 dienen auch als visuelle Marker anhand derer festgestellt werden kann, wann die Vorrichtung einsatzbereit ist. Wenn der Porenfilter 36 aufgrund des Drucks des Knochenzementteigs 54 nach vorne gedrückt wird und dabei das Styropor 40 in der Kappe 38 zusammenpresst, wird der Porenfilter 36 durch die Öffnungen 39 sichtbar. Dadurch kann der Anwender erkennen, dass der Knochenzementteig 54 fertig gemischt in der Kartusche 1 vorliegt und damit einsatzbereit ist. Der Anwender kann zu diesem Zeitpunkt die Kappe 38 mit dem Porenfilter 36 abschrauben und das Applikatorrohr 66 auf das Austragsrohr 34 aufschrauben. Anschließend kann der Austragskolben 7 über den Förderkolben 6 mit der Stange 44 angetrieben werden und dadurch der Knochenzementteig 54 aus der Kartusche 1 durch das Applikatorrohr 66 ausgetrieben werden.

### Bezugszeichenliste

- 1: Kartusche
- 2: Aufnahme
- 3: Ampulle
- 4: Monomerflüssigkeit
- 5: Zementpulver
- 6: Förderkolben
- 7: Austragskolben
- 8: Befestigungsmittel / Bajonettverschluss
- 9: Zapfen / Verschlussmittel
- 12: Keil
- 14: Verbindung
- 16: Porenfilter
- 18: Filter
- 20: Belüftungsöffnung
- 26: Dichtung
- 28: Dichtung
- 34: Austragsrohr
- 36: Porenfilter
- 37: Vertiefung
- 38: Kappe
- 39: Öffnung
- 40: Schaumstoff
- 42: Flügel
- 43: Auspressvorrichtung / Kartuschenpistole
- 44: Stange
- 46: Teller
- 48: Gegenbefestigungsmittel / Bajonettverschluss
- 50: Lagerung
- 52: Splitter
- 54: Knochenzementteig / Gemisch
- 66: Applikatorrohr

## Patentansprüche

1. Vorrichtung zum Herstellen eines Knochenzementteigs (54) aus einer Monomerflüssigkeit (4) und einem Zementpulver (5) als Ausgangskomponenten des Knochenzementteigs (54) und zum Austragen des gemischten Knochenzementteigs (54), die Vorrichtung aufweisend
eine Kartusche (1) mit einem zylindrischen Innenraum, wobei der Innenraum der Kartusche (1) an der Vorderseite bis auf eine Austragsöffnung zum Austreiben des Knochenzementteigs (54) verschlossen ist, wobei im Innenraum der Kartusche (1) ein in Richtung der Austragsöffnung drückbarer Austragskolben (7) angeordnet ist und wobei das Zementpulver (5) im Innenraum der Kartusche (1) zwischen der Austragsöffnung und dem Austragskolben (7) angeordnet ist, wobei
an der der Austragsöffnung zugewandten Vorderseite des Austragskolbens (7) ein Verschlussmittel (9) angeordnet ist, das die Austragsöffnung verschließt, wenn der Austragskolben (7) gegen die Vorderseite des Innenraums der Kartusche (1) gedrückt ist, wobei das Verschlussmittel (9) ein aus der Vorderseite des Austragskolbens (7) vorspringender Zapfen (9) ist, der zumindest teilweise in die Austragsöffnung einschiebbar ist, um die Austragsöffnung zu verschließen, wobei
der Zapfen (9) eine weitere Bewegung des Austragskolbens (7) in Richtung der Vorderseite der Kartusche (1) blockiert, so dass der Austragskolben (7) zumindest bereichsweise von der Vorderseite des Innenraums der Kartusche (1) beabstandet ist und ein Totvolumen in dem Innenraum der Kartusche (1) verbleibt, wenn der Austragskolben (7) gegen die Vorderseite des Innenraums der Kartusche (1) gedrückt ist.

2. Vorrichtung nach Anspruch 1, **dadurch gekennzeichnet, dass**
der Zapfen (9) abdichtend ist, insbesondere bei einem auf der Rückseite des Austragskolbens (7) wirkenden Druck.

3. Vorrichtung nach einem der vorangehenden Ansprüche, **dadurch gekennzeichnet, dass**
der Zapfen (9) eine geneigte, insbesondere konische, Dichtfläche aufweist, die gegen eine umlaufende Dichtkante oder Dichtfläche der Austragsöffnung abdichtet, wenn der Austragskolben (7) gegen die Vorderseite des Innenraums der Kartusche (1) gedrückt ist, oder die Austragsöffnung eine geneigte, insbesondere konische, Dichtfläche aufweist, die gegen eine umlaufende Dichtkante oder Dichtfläche des Zapfens (9) abdichtet, wenn der Austragskolben (7) gegen die Vorderseite des Innenraums der Kartusche (1) gedrückt ist.

4. Vorrichtung nach einem der vorangehenden Ansprüche, **dadurch gekennzeichnet, dass**
in dem Austragskolben (7) zumindest eine für die Monomerflüssigkeit (4) und Gase durchlässige aber für das Zementpulver (5) undurchlässige Verbindung (14) vorgesehen ist, die die Vorderseite des Austragskolbens (7) mit der Rückseite des Austragskolbens (7) verbindet.

5. Vorrichtung nach Anspruch 4, **dadurch gekennzeichnet, dass**
die zumindest eine Verbindung (14) im Austragskolben (7) innerhalb des Zapfens (9) in den Innenraum der Kartusche (1) mündet, wobei bevorzugt die zumindest eine Verbindung (14) durch mehrere radiale Bohrungen in der Mantelfläche des Zapfens (9) in den Innenraum der Kartusche (1) mündet.

6. Vorrichtung nach einem der vorangehenden Ansprüche, **dadurch gekennzeichnet, dass**
die Vorrichtung eine Aufnahme (2) aufweist, in der die Monomerflüssigkeit (4), insbesondere ein Monomerflüssigkeitsbehälter (3) enthaltend die Monomerflüssigkeit (4), enthalten ist, wobei die Rückseite der Kartusche (1) mit der Vorderseite der Aufnahme (2) verbunden ist, wobei bevorzugt
ein Innenraum der Aufnahme (2) und der Innenraum der Kartusche (1) über eine für die Monomerflüssigkeit (4) und für Gase durchlässige aber für das Zementpulver (5) undurchlässige Verbindung (14) miteinander verbunden sind.

7. Vorrichtung nach Anspruch 6, **dadurch gekennzeichnet, dass**
die Aufnahme (2) einen zylindrischen Innenraum aufweist, in dem die Monomerflüssigkeit (4), insbesondere ein Monomerflüssigkeitsbehälter (3) enthaltend die Monomerflüssigkeit (4), angeordnet ist.

8. Vorrichtung nach einem der Ansprüche 6 oder 7, **dadurch gekennzeichnet, dass**
in der Aufnahme (2) ein in Längsrichtung der Aufnahme (2) beweglicher Förderkolben (6) angeordnet ist, der von einer Rückseite der Aufnahme (2) aus in Richtung der Vorderseite vortreibbar ist, wobei die Monomerflüssigkeit (4), insbesondere ein Monomerflüssigkeitsbehälter (3) enthaltend die Monomerflüssigkeit (4), zwischen dem Förderkolben (6) und dem Austragskolben (7) angeordnet ist, wobei bevorzugt
an der Vorderseite des Förderkolbens (6) zumindest eine vortretende Spitze, Kante (12) und/oder Schneide zum Brechen des Monomerflüssigkeitsbehälters (3) angeordnet ist.

9. Vorrichtung nach einem der Ansprüche 6 bis 8, **dadurch gekennzeichnet, dass** in der Wandung der Aufnahme (2) zumindest eine Belüftungsöffnung (20) angeordnet ist, die den Innenraum der Aufnahme (2) mit der Umgebung verbindet, wobei bevorzugt die zumindest eine Belüftungsöffnung (20) derart dicht im Bereich des Förderkolbens (6) angeordnet ist, dass sie durch eine Bewegung des Förderkolbens (6) in Richtung der Vorderseite der Aufnahme (2) verschlossen wird, bevor ein in der Aufnahme (2) angeordneter Monomerflüssigkeitsbehälter (3), in dem die Monomerflüssigkeit (4) enthalten ist, durch die Bewegung des Förderkolbens (6) geöffnet ist.

10. Vorrichtung nach einem der Ansprüche 6 bis 9, **dadurch gekennzeichnet, dass**
die Rückseite der Kartusche (1) mit der Vorderseite der Aufnahme (2) derart verbunden ist, dass der Innenraum der Kartusche (1) mit dem Innenraum der Aufnahme (2) fluchtet.

11. Vorrichtung nach einem der vorangehenden Ansprüche, **dadurch gekennzeichnet, dass**
die Austragsöffnung an deren Vorderseite mit einem Verschluss (36), insbesondere mit einem Stopfen (36), verschlossen ist, wobei der Knochenzementteig (54) durch die Austragsöffnung aus der Kartusche (1) auspressbar ist, wenn die Austragsöffnung geöffnet ist, und wobei vorzugsweise der Verschluss (36) für Gase durchlässig und für das Zementpulver (5) undurchlässig ist, wobei bevorzugt
der Verschluss (36) an der dem Innenraum der Kartusche (1) zugewandten Rückseite eine Vertiefung (37) aufweist, in der der vorderste Teil des Zementpulvers (5) enthalten ist.

12. Vorrichtung nach einem der vorangehenden Ansprüche, **dadurch gekennzeichnet, dass**
der Querschnitt des Innenraums der Kartusche (1) maximal 16 cm² beträgt, bevorzugt maximal 5 cm² beträgt und/oder
das Volumen der Monomerflüssigkeit (4) in der Vorrichtung, insbesondere der Monomerflüssigkeit (4) in einem Monomerflüssigkeitsbehälter (3) in der Vorrichtung, mindestens so groß ist wie das Volumen der mit Luft gefüllten Zwischenräume zwischen den Zementpulverpartikeln in der Kartusche (1), bevorzugt mindestens so groß ist wie das Volumen der Flüssigkeitsleitungen (14) zwischen dem Innenraum der Kartusche (1) und dem Innenraum einer Aufnahme (2), in der die Monomerflüssigkeit (4) enthalten ist, plus das Volumen der mit Luft gefüllten Zwischenräume zwischen den Zementpulverpartikeln in der Kartusche (1).

13. Verfahren zur Herstellung eines Knochenzementteigs (54), insbesondere eines pastenförmigen Polymethylmethacrylat-Knochenzementteigs (54), wobei der Knochenzementteig (54) aus einem Zementpulver (5) und einer Monomerflüssigkeit (4) mit einer Vorrichtung zum Herstellen eines Knochenzementteigs (54) aus einer Monomerflüssigkeit (4) und einem Zementpulver (5) als Ausgangskomponenten des Knochenzementteigs (54) und zum Austragen des gemischten Knochenzementteigs (54) hergestellt wird, die Vorrichtung aufweisend
eine Kartusche (1) mit einem zylindrischen Innenraum, wobei der Innenraum der Kartusche (1) an der Vorderseite bis auf eine Austragsöffnung zum Austreiben des Knochenzementteigs (54) verschlossen ist, wobei im Innenraum der Kartusche (1) ein in Richtung der Austragsöffnung drückbarer Austragskolben (7) angeordnet ist und wobei das Zementpulver (5) im Innenraum der Kartusche (1) zwischen der Austragsöffnung und dem Austragskolben (7) angeordnet ist, wobei
an der der Austragsöffnung zugewandten Vorderseite des Austragskolbens (7) ein Verschlussmittel (9) angeordnet ist, das die Austragsöffnung verschließt, wenn der Austragskolben (7) gegen die Vorderseite des Innenraums der Kartusche (1) gedrückt ist, wobei das Verschlussmittel (9) eine weitere Bewegung des Austragskolbens (7) in Richtung der Vorderseite der Kartusche (1) blockiert, so dass der Austragskolben (7) zumindest bereichsweise von der Vorderseite des Innenraums der Kartusche (1) beabstandet ist und ein Totvolumen in dem Innenraum der Kartusche (1) verbleibt, wenn der Austragskolben (7) gegen die Vorderseite des Innenraums der Kartusche (1) gedrückt ist, das Verfahren umfassend die folgenden nacheinander ablaufenden Schritte:
a) Einsetzen der Vorrichtung in eine Auspressvorrichtung (43), die Auspressvorrichtung (43) aufweisend eine axial vortreibbare Stange (44), und Eindrücken der Monomerflüssigkeit (4) in den Innenraum der Kartusche (1), so dass sich die Monomerflüssigkeit (4) mit dem Zementpulver (5) mischt,
b) der Austragskolben (7) wird mit der Stange (44) in Richtung der Austragsöffnung der Kartusche (1) vorgetrieben, wobei durch die Bewegung des Austragskolbens (7) die Mischung aus dem Zementpulver (5) und der Monomerflüssigkeit (4) aus der Kartusche (1) als Knochenzementteig (54) aus der Vorrichtung ausgetrieben wird, und
c) der Austragskolben (7) auf die Vorderseite der Kartusche (1) trifft, wobei das Verschlussmittel (9) die Austragsöffnung verschließt, eine weitere Bewegung des Austragskolbens (7) in Richtung der Austragsöffnung blockiert wird und eine Restmenge der Mischung in dem Totvolumen im Innenraum der Kartusche (1) verbleibt.

14. Verfahren nach Anspruch 13, **dadurch gekennzeichnet, dass**
in Schritt a) zuerst das Einsetzen der Vorrichtung in die Auspressvorrichtung (43) erfolgt, daran anschließend ein Förderkolben (6), der innerhalb einer an der Rückseite der Kartusche (1) angeordneten Aufnahme (2) an der Rückseite der Aufnahme (2) beweglich gelagert ist, mit der Stange (44) in Richtung der Kartusche (1) vorgetrieben wird, wobei durch die Bewegung des Förderkolbens (6) ein Monomerflüssigkeitsbehälter (3), in dem die Monomerflüssigkeit (4) enthalten ist, geöffnet wird und die Monomerflüssigkeit (4) aus der Aufnahme (2) in die Kartusche (1) gepresst wird, wobei im Innenraum der Kartusche (1) sich das Zementpulver (5) mit der Monomerflüssigkeit (4) mischt, und/oder
in Schritt a) die Monomerflüssigkeit (4) durch zumindest eine für das Zementpulver (5) undurchlässige aber für Gase und die Monomerflüssigkeit (4) durchlässige Verbindung (14) im Austragskolben (7) in die Kartusche (1) gepresst wird, vorzugsweise durch eine Bewegung eines Förderkolbens (6), der mit der Stange (44) der Auspressvorrichtung (43) angetrieben wird, in die Kartusche (1) gepresst wird.

15. Verfahren nach einem der Ansprüche 13 oder 14, **dadurch gekennzeichnet, dass**
in Schritt b) durch den auf die Mischung (54) des Zementpulvers (5) mit der Monomerflüssigkeit (4) wirkenden Druck ein Verschluss (36), insbesondere ein Porenfilter (36), in einer Austragsöffnung an der Vorderseite der Kartusche (1) bewegt oder hervorgedrückt wird, wobei bevorzugt hierauf der Verschluss (36) aus der Austragsöffnung entfernt wird und besonders bevorzugt danach ein Applikationsrohr (66) an der Vorderseite der Kartusche (1) befestigt wird.

16. Verfahren nach einem der Ansprüche 13 bis 15, **dadurch gekennzeichnet, dass**
in Schritt c) die Austragsöffnung mit dem Verschlussmittel (9) aufgrund des auf die Rückseite des Austragskolbens (7) wirkenden Drucks von der Stange (44) der Auspressvorrichtung (43) abgedichtet wird.

## Claims

1. A device for producing a bone cement paste (54) from a monomer liquid (4) and a cement powder (5) as parent components of the bone cement paste (54) and for delivering the mixed bone cement paste (54), the device having
a cartridge (1) having a cylindrical interior, wherein the interior of the cartridge (1) is closed on the front side except for a delivery opening for expelling the bone cement paste (54), wherein a delivery plunger (7), which is pushable in the direction of the delivery opening, is arranged in the interior of the cartridge (1) and wherein the cement powder (5) is arranged in the interior of the cartridge (1), between the delivery opening and the delivery plunger (7), wherein
a closure means (9) is arranged on the front side, facing the delivery opening, of the delivery plunger (7), which closure means (9) closes the delivery opening when the delivery plunger (7) is pressed against the front side of the interior of the cartridge (1), wherein the closure means (9) is a pin (9) projecting from the front side of the delivery plunger (7) which is slideable into the delivery opening, at least in part, in order to close the delivery opening, wherein
the pin (9) blocks a further movement of the delivery plunger (7) in the direction of the front side of the cartridge (1) so that the delivery plunger (7) is spaced from the front side of the interior of the cartridge (1) at least in some areas and a dead volume remains in the interior of the cartridge (1) when the delivery plunger (7) is pressed against the front side of the interior of the cartridge (1).

2. The device according to claim 1, **characterized in that**
the pin (9) forms a seal, in particular when a pressure is exerted on the back side of the delivery plunger (7).

3. The device according to any one of the preceding claims, **characterized in that**
the pin (9) has an inclined, in particular conical, sealing face which forms a seal against a circumferential sealing edge or sealing face of the delivery opening when the delivery plunger (7) is pressed against the front side of the interior of the cartridge (1), or the delivery opening has an inclined, in particular conical, sealing face which forms a seal against a circumferential sealing edge or sealing face of the pin (9) when the delivery plunger (7) is pressed against the front side of the interior of the cartridge (1).

4. The device according to any one of the preceding claims, **characterized in that**
at least one connection (14), which is permeable to the monomer liquid (4) and gases but impermeable to the cement powder (5), is provided in the delivery plunger (7), which at least one connection (14) connects the front side of the delivery plunger (7) to the back side of the delivery plunger (7).

5. The device according to Claim 4, **characterized in that**
the at least one connection (14) in the delivery plunger (7) leads into the interior of the cartridge (1) within the pin (9), wherein preferably the at least one connection (14) leads into the interior of the cartridge (1) through a plurality of radial bores in the lateral surface of the pin (9).

6. The device according to any one of the preceding claims, **characterized in that**
the device has a receptacle (2) in which the monomer liquid (4), in particular a monomer liquid container (3) containing the monomer liquid (4), is contained, wherein the back side of the cartridge (1) is connected to the front side of the receptacle (2), wherein preferably
an interior of the receptacle (2) and the interior of the cartridge (1) are connected to one another via a connection (14) which is permeable to the monomer liquid (4) and to gases but impermeable to the cement powder (5).

7. The device according to Claim 6, **characterized in that**
the receptacle (2) has a cylindrical interior in which the monomer liquid (4), in particular a monomer liquid container (3) containing the monomer liquid (4), is arranged.

8. The device according to any one of Claims 6 or 7, **characterized in that**
a feed plunger (6) is arranged in the receptacle (2), the feed plunger (6) being movable in the longitudinal direction of the receptacle (2) and being drivable from a back side of the receptacle (2) in the direction of the front side, wherein the monomer liquid (4), in particular a monomer liquid container (3) containing the monomer liquid (4), is arranged between the feed plunger (6) and the delivery plunger (7), wherein preferably
at least one protruding tip, edge (12) and/or blade for breaking the monomer liquid container (3) is arranged on the front side of the feed plunger (6).

9. The device according to any one of Claims 6 to 8, **characterized in that**
at least one ventilation opening (20) is arranged in the wall of the receptacle (2), which ventilation opening connects the interior of the receptacle (2) to the environment, wherein preferably
the at least one ventilation opening (20) is arranged closely in the region of the feed plunger (6) in such a way that it is closed by a movement of the feed plunger (6) in the direction of the front side of the receptacle (2) before a monomer liquid container (3), which is arranged in the receptacle (2) and in which the monomer liquid (4) is contained, is opened by the movement of the feed plunger (6).

10. The device according to any one of Claims 6 to 9, **characterized in that**
the back side of the cartridge (1) is connected to the front side of the receptacle (2) in such a way that the interior of the cartridge (1) is flush with the interior of the receptacle (2).

11. The device according to any one of the preceding claims, **characterized in that**
the delivery opening is closed on its front side by a closure (36), in particular by a plug (36), wherein the bone cement paste (54) is pressable out of the cartridge (1) through the delivery opening when the delivery opening is open, and wherein the closure (36) is preferably permeable to gases and impermeable to the cement powder (5), wherein preferably
the closure (36) has an indentation (37) on the back side facing the interior of the cartridge (1), in which indentation the foremost part of the cement powder (5) is contained.

12. The device according to any one of the preceding claims, **characterized in that**
the cross-section of the interior of the cartridge (1) is a maximum of 16 cm², preferably a maximum of 5 cm², and/or
the volume of the monomer liquid (4) in the device, in particular the monomer liquid (4) in a monomer liquid container (3) in the device, is at least as high as the volume of the air-filled clearances between the cement powder particles in the cartridge (1), preferably at least as high as the volume of the liquid lines (14) between the interior of the cartridge (1) and the interior of a receptacle (2) in which the monomer liquid (4) is contained plus the volume of the air-filled clearances between the cement powder particles in the cartridge (1).

13. A method for producing a bone cement paste (54), in particular a paste-like polymethyl methacrylate bone cement paste (54), wherein the bone cement paste (54) is produced from a cement powder (5) and a monomer liquid (4) by a device for producing a bone cement paste (54) from a monomer liquid (4) and a cement powder (5) as parent components of the bone cement paste (54) and for delivering the mixed bone cement paste (54), the device having
a cartridge (1) having a cylindrical interior, wherein the interior of the cartridge (1) is closed on the front side except for a delivery opening for expelling the bone cement paste (54), wherein a delivery plunger (7), which is pushable in the direction of the delivery opening, is arranged in the interior of the cartridge (1) and wherein the cement powder (5) is arranged in the interior of the cartridge (1), between the delivery opening and the delivery plunger (7), wherein
a closure means (9) is arranged on the front side, facing the delivery opening, of the delivery plunger (7), which closure means (9) closes the delivery opening when the delivery plunger (7) is pressed against the front side of the interior of the cartridge (1), wherein the closure means (9) blocks a further movement of the delivery plunger (7) in the direction of the front side of the cartridge (1) so that the delivery plunger (7) is spaced from the front side of the interior of the cartridge (1) at least in some areas and a dead volume remains in the interior of the cartridge (1) when the delivery plunger (7) is pressed against the front side of the interior of the cartridge (1), the method comprising the following sequential steps:
a) inserting the device into a pressing-out device (43), the pressing-out device (43) having an axially drivable rod (44), and pushing the monomer liquid (4) into the interior of the cartridge (1) such that the monomer liquid (4) mixes with the cement powder (5),
b) the delivery plunger (7) is driven by the rod (44) in the direction of the delivery opening of the cartridge (1), wherein, as a result of the movement of the delivery plunger (7), the mixture of the cement powder (5) and the monomer liquid (4) from the cartridge (1) is expelled from the device as bone cement paste (54), and
c) the delivery plunger (7) meets the front side of the cartridge (1), wherein the closure means (9) closes the delivery opening, a further movement of the delivery plunger (7) in the direction of the delivery opening is blocked and a residual quantity of the mixture remains in the dead volume in the interior of the cartridge (1).

14. The method according to Claim 13, **characterized in that**
in step a), firstly the insertion of the device into the pressing-out device (43) takes place, after which a feed plunger (6), which is mounted inside a receptacle (2) arranged on the back side of the cartridge (1) such that it is movable inside the receptacle (2), is driven by the rod (44) in the direction of the cartridge (1), wherein, as a result of the movement of the feed plunger (6), a monomer liquid container (3) in which the monomer liquid (4) is contained is opened and the monomer liquid (4) is pressed out of the receptacle (2) into the cartridge (1), wherein the cement powder (5) mixes with the monomer liquid (4) in the interior of the cartridge (1), and/or
in step a), the monomer liquid (4) is pressed into the cartridge (1) through at least one connection (14) in the delivery plunger (7), which is impermeable to the cement powder (5) but permeable to gases and the monomer liquid (4), preferably pressed into the cartridge (1) by a movement of a feed plunger (6) which is driven by the rod (44) of the pressing-out device (43).

15. The method according to any one of Claims 13 or 14, **characterized in that**
in step b), due to the pressure exerted on the mixture (54) of the cement powder (5) with the monomer liquid (4), a closure (36), in particular a porous filter (36), is moved or pushed forward in a delivery opening on the front side of the cartridge (1), whereupon the closure (36) is preferably removed from the delivery opening and an application tube (66) is then particularly preferably fastened to the front side of the cartridge (1).

16. The method according to any one of Claims 13 to 15, **characterized in that**
in step c), the delivery opening is sealed by the closure means (9) owing to the pressure exerted on the back side of the delivery plunger (7) by the rod (44) of the pressing-out device (43).

## Revendications

1. Dispositif permettant la préparation d'une pâte de ciment osseux (54) à partir d'un liquide monomère (4) et d'une poudre de ciment (5) comme composants de départ de la pâte de ciment osseux (54) et la distribution de la pâte de ciment osseux (54) mélangée, le dispositif présentant
une cartouche (1) comportant un espace intérieur cylindrique, dans lequel l'espace intérieur de la cartouche (1) est fermé sur le côté frontal, à l'exception d'une ouverture de distribution pour l'évacuation de la pâte de ciment osseux (54), dans lequel, dans l'espace intérieur de la cartouche (1), il est disposé un piston de distribution (7) pouvant être pressé en direction de l'ouverture de distribution et dans lequel la poudre de ciment (5) dans l'espace intérieur de la cartouche (1) est disposée entre l'ouverture de distribution et le piston de distribution (7), dans lequel
sur le côté frontal du piston de distribution (7) faisant face à l'ouverture de distribution, il est disposé un moyen de fermeture (9) qui ferme l'ouverture de distribution lorsque le piston de distribution (7) est pressé contre le côté frontal de l'espace intérieur de la cartouche (1), dans lequel le moyen de fermeture (9) est un tourillon (9) faisant saillie depuis le côté frontal du piston de distribution (7), lequel tourillon peut être inséré au moins partiellement dans l'ouverture de distribution pour fermer l'ouverture de distribution, dans lequel
le tourillon (9) bloque un mouvement supplémentaire du piston de distribution (7) en direction du côté frontal de la cartouche (1), de sorte que le piston de distribution (7) est espacé, au moins dans certaines régions, du côté frontal de l'espace intérieur de la cartouche (1) et qu'un volume mort reste dans l'espace intérieur de la cartouche (1) lorsque le piston de distribution (7) est pressé contre le côté frontal de l'espace intérieur de la cartouche (1).

2. Dispositif selon la revendication 1, **caractérisé en ce que**
le tourillon (9) est étanche, en particulier en cas de pression agissant sur le côté arrière du piston de distribution (7).

3. Dispositif selon l'une des revendications précédentes, **caractérisé en ce que**
le tourillon (9) présente une surface d'étanchéité inclinée, en particulier conique, qui assure l'étanchéité par rapport à une arête d'étanchéité ou une surface d'étanchéité périphérique de l'ouverture de distribution lorsque le piston de distribution (7) est pressé contre le côté frontal de l'espace intérieur de la cartouche (1), ou l'ouverture de distribution présente une surface d'étanchéité inclinée, en particulier conique, qui assure l'étanchéité par rapport à une arête d'étanchéité ou une surface d'étanchéité périphérique du tourillon (9) lorsque le piston de distribution (7) est pressé contre le côté frontal de l'espace intérieur de la cartouche (1).

4. Dispositif selon l'une des revendications précédentes, **caractérisé en ce que**
il est prévu, dans le piston de distribution (7), au moins un raccord (14) non étanche au liquide monomère (4) et aux gaz mais étanche à la poudre de ciment (5), lequel raccord relie le côté frontal du piston de distribution (7) au côté arrière du piston de distribution (7).

5. Dispositif selon la revendication 4, **caractérisé en ce que**
l'au moins un raccord (14) dans le piston de distribution (7) à l'intérieur du tourillon (9) débouche dans l'espace intérieur de la cartouche (1), dans lequel préférablement l'au moins un raccord (14) débouche dans l'espace intérieur de la cartouche (1) à travers plusieurs alésages radiaux dans la surface d'enveloppe du tourillon (9).

6. Dispositif selon l'une des revendications précédentes, **caractérisé en ce que**
le dispositif présente un logement (2) dans lequel est contenu le liquide monomère (4), en particulier un récipient de liquide monomère (3) contenant le liquide monomère (4), dans lequel le côté arrière de la cartouche (1) est raccordé au côté frontal du logement (2), dans lequel préférablement
un espace intérieur du logement (2) et l'espace intérieur de la cartouche (1) sont raccordés entre eux par l'intermédiaire d'un raccord (14) non étanche au liquide monomère (4) et aux gaz mais étanche à la poudre de ciment (5).

7. Dispositif selon la revendication 6, **caractérisé en ce que**
le logement (2) présente un espace intérieur cylindrique dans lequel est disposé le liquide monomère (4), en particulier un récipient de liquide monomère (3) contenant le liquide monomère (4).

8. Dispositif selon l'une des revendications 6 ou 7, **caractérisé en ce que**
il est disposé, dans le logement (2), un piston de transport (6) mobile dans le sens longitudinal du logement (2), lequel piston de transport peut être avancé à partir d'un côté arrière du logement (2) en direction du côté frontal, dans lequel le liquide monomère (4), en particulier un récipient de liquide monomère (3) contenant le liquide monomère (4), est disposé entre le piston de transport (6) et le piston de distribution (7), dans lequel préférablement
sur le côté frontal du piston de transport (6), il est disposé au moins une pointe, une arête (12) et/ou un tranchant en saillie permettant de fracturer le récipient de liquide monomère (3).

9. Dispositif selon l'une des revendications 6 à 8, **caractérisé en ce que**
dans la paroi du logement (2), il est disposé au moins une ouverture d'aération (20) qui raccorde l'espace intérieur du logement (2) à l'environnement, dans lequel préférablement l'au moins une ouverture d'aération (20) est disposée de façon étanche dans la zone du piston de transport (6) de telle sorte qu'elle est fermée par un mouvement du piston de transport (6) en direction du côté frontal du logement (2) avant qu'un récipient de liquide monomère (3), disposé dans le logement (2) et dans lequel le liquide monomère (4) est contenu, ne soit ouvert par le mouvement du piston de transport (6).

10. Dispositif selon l'une des revendications 6 à 9, **caractérisé en ce que**
le côté arrière de la cartouche (1) est raccordé au côté frontal du logement (2) de telle sorte que l'espace intérieur de la cartouche (1) est aligné avec l'espace intérieur du logement (2).

11. Dispositif selon l'une des revendications précédentes, **caractérisé en ce que**
l'ouverture de distribution est fermée sur son côté frontal à l'aide d'une fermeture (36), en particulier à l'aide d'un bouchon (36), dans lequel la pâte de ciment osseux (54) peut être expulsée par pression hors de la cartouche (1) à travers l'ouverture de distribution lorsque l'ouverture de distribution est ouverte, et dans lequel de préférence la fermeture (36) est non étanche aux gaz et étanche à la poudre de ciment (5), dans lequel préférablement la fermeture (36) présente, sur le côté arrière faisant face à l'espace intérieur de la cartouche (1), un évidement (37) dans lequel est contenue la partie la plus en avant de la poudre de ciment (5).

12. Dispositif selon l'une des revendications précédentes, **caractérisé en ce que**
la section transversale de l'espace intérieur de la cartouche (1) est au maximum de 16 cm², préférablement au maximum de 5 cm², et/ou
le volume du liquide monomère (4) dans le dispositif, en particulier du liquide monomère (4) dans un récipient de liquide monomère (3) dans le dispositif, est au moins aussi important que le volume des espaces intermédiaires remplis d'air entre les particules de poudre de ciment dans la cartouche (1), préférablement au moins aussi important que le volume des conduites de liquide (14) entre l'espace intérieur de la cartouche (1) et l'espace intérieur d'un logement (2) dans lequel le liquide monomère (4) est contenu, plus le volume des espaces intermédiaires remplis d'air entre les particules de poudre de ciment dans la cartouche (1).

13. Procédé permettant la préparation d'une pâte de ciment osseux (54), en particulier d'une pâte de ciment osseux de polyméthacrylate de méthyle (54) pâteuse, dans lequel la pâte de ciment osseux (54) est préparée à partir d'une poudre de ciment (5) et d'un liquide monomère (4) à l'aide d'un dispositif permettant la préparation d'une pâte de ciment osseux (54) à partir d'un liquide monomère (4) et d'une poudre de ciment (5) comme composants de départ de la pâte de ciment osseux (54) et la distribution de la pâte de ciment osseux (54) mélangée, le dispositif présentant
une cartouche (1) comportant un espace intérieur cylindrique, dans lequel l'espace intérieur de la cartouche (1) est fermé sur le côté frontal, à l'exception d'une ouverture de distribution pour l'évacuation de la pâte de ciment osseux (54), dans lequel, dans l'espace intérieur de la cartouche (1), il est disposé un piston de distribution (7) pouvant être pressé en direction de l'ouverture de distribution et dans lequel la poudre de ciment (5) dans l'espace intérieur de la cartouche (1) est disposée entre l'ouverture de distribution et le piston de distribution (7), dans lequel
un moyen de fermeture (9) qui ferme l'ouverture de distribution est disposé sur le côté frontal du piston de distribution (7) faisant face à l'ouverture de distribution lorsque le piston de distribution (7) est pressé contre le côté frontal de l'espace intérieur de la cartouche (1), dans lequel le moyen de fermeture (9) bloque un mouvement supplémentaire du piston de distribution (7) en direction du côté frontal de la cartouche (1), de sorte que le piston de distribution (7) est espacé, au moins dans certaines régions, du côté frontal de l'espace intérieur de la cartouche (1) et qu'un volume mort reste dans l'espace intérieur de la cartouche (1) lorsque le piston de distribution (7) est pressé contre le côté frontal de l'espace intérieur de la cartouche (1), le procédé comprenant les étapes successives suivantes :
a) mise en place du dispositif dans un dispositif d'expulsion par pression (43), le dispositif d'expulsion par pression (43) présentant une tige (44) pouvant être avancée axialement, et enfoncement du liquide monomère (4) dans l'espace intérieur de la cartouche (1), de sorte que le liquide monomère (4) se mélange avec la poudre de ciment (5),
b) le piston de distribution (7) est avancé avec la tige (44) en direction de l'ouverture de distribution de la cartouche (1), dans lequel, au moyen du mouvement du piston de distribution (7), le mélange composé de la poudre de ciment (5) et du liquide monomère (4) est expulsé de la cartouche (1) à partir du dispositif comme pâte de ciment osseux (54), et
c) le piston de distribution (7) frappe le côté frontal de la cartouche (1), dans lequel le moyen de fermeture (9) ferme l'ouverture de distribution, un mouvement supplémentaire du piston de distribution (7) en direction de l'ouverture de distribution est bloqué et une quantité restante du mélange reste dans le volume mort dans l'espace intérieur de la cartouche (1).

14. Procédé selon la revendication 13, **caractérisé en ce que**
à l'étape a), la mise en place du dispositif dans le dispositif d'expulsion par pression (43) a d'abord lieu, puis un piston de transport (6), lequel est monté mobile à l'intérieur d'un logement (2) disposé sur le côté arrière de la cartouche (1) sur le côté arrière du logement (2), est avancé avec la tige (44) en direction de la cartouche (1), dans lequel, au moyen du mouvement du piston de transport (6), un récipient de liquide monomère (3), dans lequel est contenu le liquide monomère (4), est ouvert et le liquide monomère (4) provenant du logement (2) est pressé dans la cartouche (1), dans lequel, dans l'espace intérieur de la cartouche (1), la poudre de ciment (5) se mélange au liquide monomère (4), et/ou
à l'étape a), le liquide monomère (4) est pressé dans le piston de distribution (7) dans la cartouche (1) par au moins un raccord (14) étanche à la poudre de ciment (5) mais non étanche aux gaz et au liquide monomère (4), de préférence est pressé dans la cartouche (1) par un mouvement d'un piston de transport (6) entraîné avec la tige (44) du dispositif d'expulsion par pression (43).

15. Procédé selon l'une des revendications 13 ou 14, **caractérisé en ce que**
à l'étape b), sous l'action de la pression agissant sur le mélange (54) de la poudre de ciment (5) avec le liquide monomère (4), une fermeture (36), en particulier un filtre poreux (36), est déplacé ou enfoncé dans une ouverture de distribution sur le côté frontal de la cartouche (1), dans lequel préférablement la fermeture (36) est ensuite retirée de l'ouverture de distribution et de manière particulièrement préférée, après cela, un tube d'application (66) est fixé sur le côté frontal de la cartouche (1).

16. Procédé selon l'une des revendications 13 à 15, **caractérisé en ce que**
à l'étape c), l'ouverture de distribution est rendue étanche à l'aide du moyen de fermeture (9) en raison de la pression de la tige (44) du dispositif d'expulsion par pression (43), laquelle pression agit sur le côté arrière du piston de distribution (7).
